# EUROPEAN PATENT APPLICATION

(11) **EP 4 559 486 A1**
(43) Date of publication of application: **28.05.2025**
(21) Application number: 23211132.8
(22) Date of filing: 21.11.2023
(51) Int. Cl.: A61L 27/18, A61L 27/26, A61L 27/50, A61L 27/58, A61L 31/04, A61L 31/06, A61L 31/14

(54) **A DEVICE FOR REPAIR SURGERY OF CYLINDRICAL ORGANS, PARTICULARLY RUPTURED TENDONS, AND METHOD OF PRODUCING SUCH DEVICE**

(71) Applicant: Universität Zürich, 8006 Zürich (CH)
(72) Inventor: Miescher, Iris, 8610 Uster (CH); Calcagni, Maurizio, 8008 Zürich (CH); Buschmann, Johanna, 8008 Zürich (CH)
(74) Representative: Schmauder & Partner AG Patent- & Markenanwälte VSP

(57) **Abstract**

A device for repair surgery of cylindrical organs, particularly of ruptured tendons, is configured as a tubular sheath (T) made of a mesh of elastic fibers formed by electrospinning biocompatible and biodegradable polymers. The tubular sheath has a first, inner wall surface and a second,outer wall surface substantially parallel thereto, with said first wall surface being comparatively rough (W_{R}) and said second wall surface being comparatively smooth (Ws).

## Description

### Field of the Invention

The present invention relates to a device for repair surgery of cylindrical organs, particularly ruptured tendons. Moreover, the invention relates to a method of producing such a device.

### Background of the Invention

Tendon ruptures constitute a major part of musculoskeletal injuries, with Achilles tendon ruptures as one of the most frequently ruptured tendons in the human body [A1]. Functional loss of the repaired tendons is due to two major drawbacks, (i) adhesion formation to the surrounding tissue, resulting in a reduced range of motion and (ii) insufficient mechanical strength acquired during initial tendon healing, leading to re-rupture [A1]. Due to limited tendon vascularity and innervation, the natural healing of the tendon is inefficient [A2] and therapeutic repair options include autografts, allografts, xenografts, suture techniques and tendon prostheses [A3].

A regenerative engineering approach addressing the major drawbacks in Achilles tendon rupture, has been explored before by developing a reversibly expandable electrospun polymer tube, made from a biodegradable co-block polymer, a polyester urethane named DegraPol^{®} (DP) [A4], see also WO 2013/026937 A1. Electrospun tube made of a newly synthesized type of DP, which is softer and more elastic, surgeon friendly and better suited for tendon rupture repair [A4], compared to the already established classic DP [A5] has been applied over the wound site after conventional tendon repair. It has been shown that is does not evoke an adverse cellular response and that it significantly minimized peritendinous adhesion formation in a rabbit model [A6]. Using this newly synthesized DP for production of reversibly expendable electrospun scaffolds/tubes offers a big advantage over other electrospun polymers like poly(lactide-co-glycolic acid) (PLGA) or poly(caprolactone) (PCL), that exhibit considerably low strain at break [A7].

To address the issue of insufficient strength at the tendon rupture repair site and to further develop the elastic DP tube as an implant for tendon rupture repair, it has been explored as a carrier system for growth factor(s), more specifically platelet-derived growth factor - BB (PDGF-BB). This approach aims to provide a bioactive scaffold that can locally promote and accelerate tendon healing. Administration of biological molecules like growth factors has been proposed and studied in aiding the recapitulation of the native tendon function after injury [A8]. More specifically, PDGF-BB has been shown to affect matrix remodeling, increase collagen synthesis and cell proliferation [A9], thus its local delivery might also affect the biomechanical strength of the repaired tendons.

WO 2017/137602 A1 discloses a device for repair surgery of cylindrical organs, particularly ruptured tendons, is configured as a tubular sheath (T) made of a biocompatible and biodegradable polymer. The tubular sheath comprises an elastic fiber mesh formed by electrospinning of said polymer and has an inner wall surface and an outer wall surface substantially parallel thereto. One of said wall surfaces is comparatively rough (W_{R}) and the other one of said wall surfaces is comparatively smooth (Ws), with the tubular sheath having a Young elasticity modulus of about 0.1 to about 4 MPa and an elongation at break of about 50 to about 1'000 %. The polymer is a biodegradable polyester urethane block copolymer with poly-hydroxy-butyrate as a hard segment and ε-caprolactone as a soft segment. elastic fibers comprise first fibers consisting of a first one of said polymers in neat form and second fibers consisting of a second one of said polymers with an admixture of a therapeutic agent for stimulating regrowth processes of a predetermined cylindrical organ, said tubular sheath comprising a first tubular region adjacent to said first wall and a second tubular region adjacent to said second wall, said first tubular region being formed of said first fibers and said second tubular region being formed of said second fibers.

### Summary of the Invention

It is an object of the present invention to provide a still further improved device for repair surgery of cylindrical organs, particularly ruptured tendons.

These objects are achieved by a device for repair surgery of cylindrical organs, particularly ruptured tendons, as defined in claim 1.

The device is configured as a tubular sheath (T) made of a mesh of elastic fibers formed by electrospinning biocompatible and biodegradable polymers, said tubular sheath having a Young elasticity modulus of about 0.1 to about 4 MPa and a strain at break of about 50 to about 1'000 %. The tubular sheath has a first, inner wall surface (W_{I}) and a second, outer wall surface (Wo) substantially parallel thereto. The first, inner wall surface is comparatively rough (W_{R}) and the second, outer wall surface is comparatively smooth (Ws). The tubular sheath (T) comprises a first tubular layer adjacent to said first, inner wall surface and a second tubular layer adjacent to said second, outer wall surface, said first tubular layer being formed of first polymeric fibers and said second tubular layer being formed by second polymeric fibers.

According to the invention, the first polymeric fibers are formed from degrapol (DP) and the second fibers are formed from a blend of high molecular weight hyaluronic acid (HA) and polyethylene oxide (PEO).

While it is contemplated that the device may be used for repair surgery of a whole variety of cylindrical organs such as nerves, blood vessels and certain muscles in humans or other animals, particularly mammals, it is particularly useful for repair surgery of ruptured tendons in humans. In the present context the term "cylindrical" shall be understood as "having substantially cylindrical symmetry" in line with the fact that human and animal organs do not exhibit perfect cylindricity in the geometrical sense both because of inherent slight irregularities of their shape and variations in cross sectional size, but also because of their deformability.

The term "polymer in neat form" shall be understood as a polymeric material as defined elsewhere without any admixture of the therapeutic agent selected for stimulating regrowth processes in the selected organ. It is not excluded, however, that the first tubular region could contain some other type of therapeutic agents to be delivered in an outwards direction, i.e. to a body region peripherally surrounding the ruptured organ.

The term "polymer with an admixture of a therapeutic agent for stimulating regrowth processes" shall be understood in a broad sense so as to include various types of loading the therapeutic agent onto or into a polymeric fiber.

The terms "biocompatible" and "biodegradable" are generally known in the field of surgery. Synthetic biocompatible and bioresorbable polymers are becoming increasingly popular for surgical applications either as tissue engineered artificial grafts and/ or as bioactive carrier devices delivering growth factors [A10], cytokines and other bioactive substances [A11], [A12], [A13]. Tendon grafts can also be seeded with stem cells improving the early healing process [A14]. The advantage of such polymers is that their mechanical properties as well as their degradation rates can be controlled and adjusted for specific medical applications [A15], [A16], [A5], [A17].
Moreover, tissue integration can be regulated by porosity and architecture of the material used for grafting [A18].

The technique of forming a tubular sheath comprising an elastic polymeric fiber mesh by electrospinning is generally known (see, e.g. US 2011/0039101 A1). As the fiber mesh is formed on a cylindrical or conical target with a smooth surface, the resulting sheath has a comparatively smooth surface on its inner wall adjacent the target and a comparatively rough outer surface on its outer wall. For example, the smooth surface will have a dynamic friction coefficient of about 0.85 whereas the rough surface will have a dynamic friction coefficient of about 1.05. As will be explained in more detail further below, this difference in surface roughness can be exploited in repair surgery by first everting the tubular sheath so as to have the rough surface as the inner wall. This allows for firm contact of the sheath with the cylindrical organ to be repaired and reduces the friction between the outer wall and any surrounding tissue, thereby improving mobility of the repaired organ, for example gliding of a tendon in the tendon sheath.

By virtue of its configuration of a double-layered electrospun tube which, when applied on a ruptured organ site, has a comparatively rough bioactive inner layer and a comparatively smooth outer layer acting as a physical barrier towards the surrounding tissue, the device of the present invention provides for a substantively improved healing process.
Inventors have discovered that by configuring the outer layer from a blend of high molecular weight hyaluronic acid (HA) and polyethylene oxide (PEO) leads to an unexpectedly advantageous performance in terms of a lubrication-like effect. Formation of unwanted adhesion from surrounding tissue is substantially avoided.

In particular, a tubular implant material based on a 2-layer electrospun mesh for improvement of tendon repair by anti-adhesion effects. One layer consists of DegraPol^{®} and one layer of a blend of high molecular weight hyaluronic acid (1.01 - 1.8 MDa) (HA) and polyeth-ylene oxide (600 kDa PEO) in a ratio of 1:1 as a blend.

By having a Young elasticity modulus of about 0.1 to about 4 MPa and a strain at break of about 50 to about 1'000 %, the tubular sheath can be readily expanded when being placed on the repaired region and it can subsequently provide a long lasting radially inward directed pressure that contributes to the improved healing process.

The diameter of the tubular sheath will be selected according to the size of the organ to be repaired. In general, it will be chosen to be slightly narrower than the outer diameter of the organ to be repaired, so that the tubular sheath can be applied onto the relevant organ region after slight radial expansion. For the repair of human tendons, the tubular shell will have a diameter in the range of about 1 to about 5 mm.

Advantageous embodiments of the invention are defined in the dependent claims.

According to one embodiment (claim 2), the first polymeric fibers are formed from degrapol (DP) with an admixture of a therapeutic agent (GA) for stimulating regrowth processes of a predetermined cylindrical organ. When the fiber material of the inner wall slowly decomposes, typically on a time scale of a few weeks, the therapeutic agent contained therein is gradually released and delivered to the repaired organ region.

Acording to a further emodiment (claim 3), the first tubular layer and the second tubular layer each form about one half of the tubular sheath's wall thickness.

It is contemplated that the biocompatible and biodegradable polymers used to form the first fibers and the second fibers, respectively, may be different polymeric species. In particular, the polymer used to form the first fibers, henceforth also called "first polymer", may be selected to optimize the release of any therapeutic agent to be delivered and to optimize mechanical properties of the device. In contrast, the polymer used to form the second fibers, henceforth also called "second polymer", may be selected to optimize the anti-adhesive properties of the device.

According to certain embodiments, the first polymer is a biodegradable polyester urethane block copolymer with poly-hydroxy-butyrate as a hard segment and ε-caprolactone as a soft segment. Such polymers are known and can be purchased as DegraPol^{®} from ab medica s.p.a., Italy. Degrapol has been shown to be biocompatible for fibroblasts, osteoblasts and tenocytes in vitro; moreover, it is biodegradable as well as cyto- and hemocompatible [A15], [A5]. The degradation rate can be adjusted in the range from a few weeks up to a few years. It is contemplated that different subtypes of the above mentioned polyester urethane block copolymer can be used to form the first and second polymer fibers.

A particularly preferred embodiment involves a selected type of the above mentioned polyester urethane block copolymer, characterized by a soft segment with an average molecular weight of about 900g/mol to about 1'250 g/mol, a relative content of said soft segment of about 60 to about 75 parts by weight and a relative content of said hard segment of about 40 to about 25 parts by weight. The term "relative content" is used here because, as will be generally known, formation of such block copolymers further requires the addition of an appropriate coupling agent. In the present case, this is an isocyanate coupler. If not specifically mentioned otherwise, average molecular weights reported here are number averaged molecular weights Mₙ, which can be determined e.g. by means of gel permeation chromatography (GPC).

According to other embodiments, the tubular sheath has a Young elasticity modulus of about 0.4 to about 2.5 MPa and a strain at break of about 200 to about 1000 %.

As already pointed out further above, the device of the present invention is generally intended for repair surgery of organs with substantially cylindrical symmetry. This also includes configurations with variable diameter. Therefore, according to one embodiment, the tubular sheath is of substantially frustoconical shape, i.e. it has a diameter that monotonously decreases in one axial direction. Typically, the half-aperture angle, i.e. the angle between the wall surface and the longitudinal axis will be in the range of about 1 to about 10°. However, the useful tubular shapes also include configurations with a nonlinear diameter variation, i.e. having a curved longitudinal section. The specific shape will be selected according to the shape of the organ to be repaired. As will be understood, the manufacture of tubular sheaths with such specific shapes by electrospinning is readily achieved by selecting an appropriately formed deposition target.

According to an advantageous embodiment, the first fibers are heterogeneous filaments having included cavities filled with said therapeutic agent. Such fibers can be produced by the method of emulsion electrospinning, in which the process is applied to an emulsion of the polymer and an aqueous solution containing the therapeutic agent. Depending on the process parameters, the agent filled cavities will have varying sizes and will be distributed randomly within the fiber. The release of therapeutic agent will begin as soon as there has been sufficient polymer degradation to expose some of the filled cavities.

According to another advantageous embodiment, the first fibers are hollow filaments having a central core filled with said therapeutic agent. With such a tubular core-shell architecture, the release of therapeutic agent will begin in a nearly step-like manner once the polymer forming the tube wall has sufficiently degraded.

The first tubular region may form about 5 to about 95 percent of the wall thickness, with the remainder being formed by the second tubular region. In this manner one can optimize the amount of therapeutic agent that is ultimately released by the device and the mechanical properties. According to one embodiment, the first tubular region and the second tubular region each form about one half of the sheath's wall thickness.

The device of the present invention is suitable for delivery of a large variety of therapeutic agents, which term shall be interpreted in a broad sense and include any agents that could have a beneficial effect on the healing process after repair surgery. In particular, the therapeutic agent can be selected from the group consisting of growth hormones, pharmaceutical agents and growth promoting cells, including stem cells. In a particularly advantageous embodiment the therapeutic agent is platelet-derived growth factor - BB or insulin-like growth factor-1 [A19].

According to another aspect of the invention, there is provided a method of producing a device as defined above, in which method said elastic fibers are formed by solution electrospinning. This technique is generally known. In contrast to melt electrospinning, in which polymer fibers are formed from a melt of the respective polymeric species, solution electrospinning is based on using a solution of the respective polymeric species in a volatile solvent. The solution is driven from a reservoir through a nozzle or needle, after the exit of which the solvent evaporates and a thread of first polymer fibers is formed.

### References

[A1] S. Thomopoulos, W. C. Parks, D. B. Rifkin, K. A. Derwin, J. Orthop. Res. 2015, 33, 832.
[A2] D. Elliot, T. Giesen, Hand clin. 2013, 29, 191.
[A3] J. C. Goh, H. W. Ouyang, S. H. Teoh, C. K. Chan, E. H. Lee, Tissue Eng. 2003, 9, S31.
[A4] a) J. Buschmann, M. Calcagni, G. M. Burgisser, E. Bonavoglia, P. Neuenschwander, V. Milleret, P. Giovanoli, J. Tissue Eng. Regen. Med. 2015, 9, 584; b) J. Buschmann, G. Meier-Burgisser, E. Bonavoglia, P. Neuenschwander, V. Milleret, P. Giovanoli, M. Calcagni, J. Tissue Eng. Regen. Med. 2013, 7, 413.
[A5] a) V. Milleret, M. Simonet, A. G. Bittermann, P. Neuenschwander, H. Hall, J. Biomed. Mat. Res. B Applied Biomater. 2009, 91, 109; V. Milleret, B. Simona, P. Neuenschwander, H. Hall, Eur. Cell Mater. 2011, 21, 286; c) S. A. Riboldi, M. Sampaolesi, P. Neuenschwander, G. Cossu, S. Mantero, Biomaterials. 2005, 26, 4606.
[A6] a) J. Buschmann, G. Puippe, G. M. Bürgisser, E. Bonavoglia, P. Giovanoli, M. Calcagni, Connect. Tissue Res. 2014, 55, 123; b) G. M. Bürgisser M. Calcagni, Müller A, E. Bonavoglia, G Fessel, J. G. Snedeker, P. Giovanoli, J. Buschmann, Biomed. Res. Int. 2014, 2014, 656240.
[A7] a) Y. Qian, Z. Zhang, L. Zheng, R. Song, Y. Zhao. J. Nanomat. 2014, 2014, 964621. b) V. Milleret, B. Simona, P. Neuenschwander, H. Hall, Eur. Cell Mater. 2011, 21, 286.
[A8] a) N. Bachl, W. Derman, L. Engebretsen, G. Goldspink, M. Kinzlbauer, H. Tschan, P. Volpi, D. Venter, B. Wessner, J. Sports Med. Phys. Fitness. 2009, 49, 346; b) L. V. Gulotta, S. A. Rodeo, Clin. Sports Med. 2009, 28, 13; c) M. A. Sandrey, J. Athl. Train. 2014, 49, 428.
[A9] a) S. Thomopoulos, M. Zaegel, R. Das, F. L. Harwood, M. J. Silva, D. Amiel, S. Sakiyama-Elbert, R. H. Gelberman, J. Orthop. Res. 2007, 25, 1358; b) S. Thomopoulos, R. Das, M. J. Silva, S. Sakiyama-Elbert, F. L. Harwood, E. Zampiakis, H. M. Kim, D. Amiel, R. H. Gelberman, J. Orthop. Res. 2009, 27, 1209; c) Uggen, J. Dines, M. McGarry, D. Grande, T. Lee, O. Limpisvasti, Arthroscopy. 2010, 26, 1456.
[A10] M. A. Costa, C. Wu, B.V. Pham, A. K. S. Chong, H. M. Pham, J. Chang, Tissue Engineering 2006, 12, 1937.
[A11] C.F. Maurus, M. K. J. Schneider, D. Schmidt, G. Zund, J.D. Seebach, Transplantation, 2006, 81, 1204.
A12] K. A. Corsi, E. M. Schwarz, D. J. Mooney and J. Huard, Journal of Orthopaedic Research, 2007, 25, 1261.
[A13] R. Bullough, T. Finnigan, A. Kay, N. Maffulli, N. R. Forsyth, Disability and Rehabilitation, 2008, 30, 1746.
[A14] A. K. S. Chong, A. D. Ang, J. C. H. Goh, J. H. P. Hui, A. Y. T. Lim, E. H. Lee, Journal of Bone and Joint Surgery, 2007, American Volume, 89A, 74.
[A15] B. Saad, Y. Kuboki, M. Welti, G. K. Uhlschmid, P. Neuenschwander, U. W. Suter, (1999). DegraPol-Foam: A degradable and highly porous polyesterurethane foam as a new substrate for bone formation. 12th World Congress of the International-Society-for-Artificial-Organs/26th Congress of the European-Society-for-Artificial-Organs, Edinburgh, Scotland, Blackwell Science Inc.
[A16] L. Durselen, M. Dauner, H. Hierlemann, H. Planck, L. E. Claes, A. Ignatius, Journal of Biomedical Materials Research, 2001, 58, 666.
[A17] S. Sahoo, S. L. Toh, J. C. H. Goh, Journal of Biomedical Materials Research Part B-Applied Biomaterials, 2010, 95B, 19.
[A18] J. A. Henry, K. Burugapalli, P. Neuenschwander, A. Pandit, Acta Biomaterialia, 2009, 5, 29.
[A19] J. Rieber, Meier-Biirgisser, G.; Miescher, I.; Weber, F. E.; Wolint, P.; Yao, Y.; Ongini, E.; Milionis, A.; Snedeker, J. G.; Calcagni, M.; et al. Int. J. Mol. Sci. 2023, 24 (12).

### Brief description of the drawings

The above mentioned and other features and objects of this invention and the manner of achieving them will become more apparent and this invention itself will be better understood by reference to the following description of various embodiments of this invention taken in conjunction with the accompanying drawings, wherein:
- Fig. 1: shows a generic device for repair surgery, in a longitudinal cross-section;
- Fig. 2: shows a first embodiment of a device for repair surgery, in a longitudinal cross-section;
- Fig. 3: shows a further embodiment of a device for repair surgery, in a longitudinal cross-sec-tion;
- Fig. 4: shows an overview of the experimental procedure, namely:
**A**) Photo and scheme of electrospun DegraPol^{®} tube with a layer of HA/PEO in a ratio 1:1 or 1:4;
**B**) In vitro experiment using rabbit tenocytes seeded on electrospun DP scaffolds with or without a layer of HA/PEO in order to investigate cell adhesion and proliferation;
**C**) Rabbit AT full laceration model, using flipped DP implants that contain a HA/PEO layer in a ratio 1:1, facing the surrounding tissue;
**D**) Material property analysis of the differently produced electrospun DP tubes (with or without HA/PEO) by Scanning electron microscope (SEM) pictures, Fourier-transform Infrared Spectroscopy (FTIR), Differential Scanning Calorimetry (DSC), static and dynamic Water Contact Angle (WCA) and mechanical test;

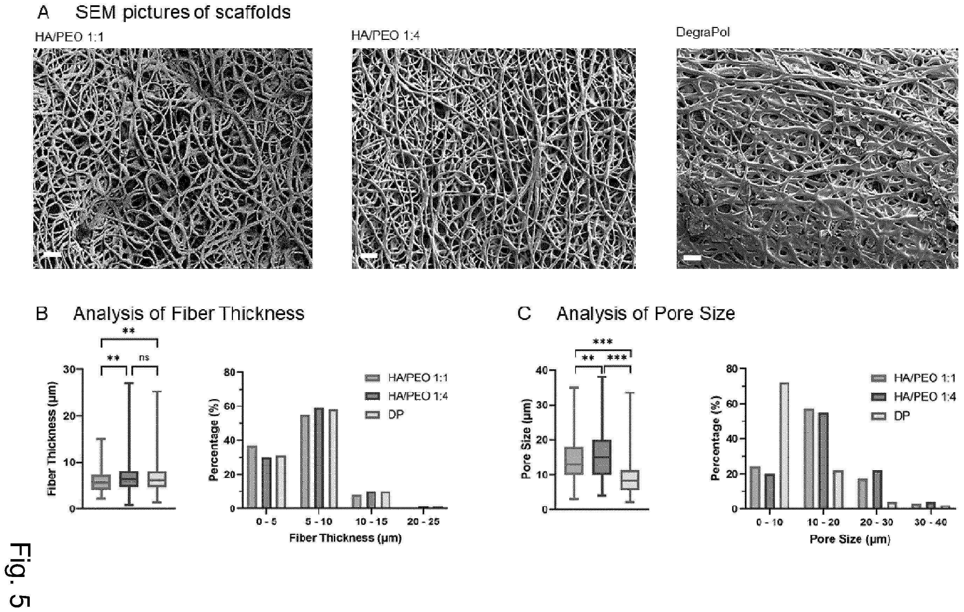

- Fig. 5: shows an analysis of scanning electron microscope images regarding fibe thickness and pore size of DP scaffolds containing a HA layer and pure DP scaffolds, namely:
**A**) SEM images of differently produced scaffolds;
**B**) analysis of the inner scaffold layer consisting of HA/PEO in a ratio 1:1 or 1:4 or pure DP scaffold, fiber thickness (B) and pore size (C) were assessed; number of tubes n = 3 for all HA containing scaffolds, n = 6 for fiber analysis of DP scaffolds and n = 3 for pore size DP scaffolds analysis; an ordinary one-way-ANOVA for each surface was performed (Tukey's multiple comparisons test) to determine significant median differences; data are shown as box and whisker plots with interquartile range and 95 % confidence interval; pvalues ≤ 0.05 were considered significant and denoted with (*), for p-values ≤ 0.01 (**), p-values ≤ 0.001 (***) and for non-significant (ns) was used; numbers of fibers analyzed to determine fiber thickness HA/PEO (1:1) n = 411; HA/PEO (1:4) n = 737; DP n = 1178. Numbers of pores measured to determine pore size HA/PEO (1:1) n = 490; HA/PEO (1:4) n = 797; DP n = 95;

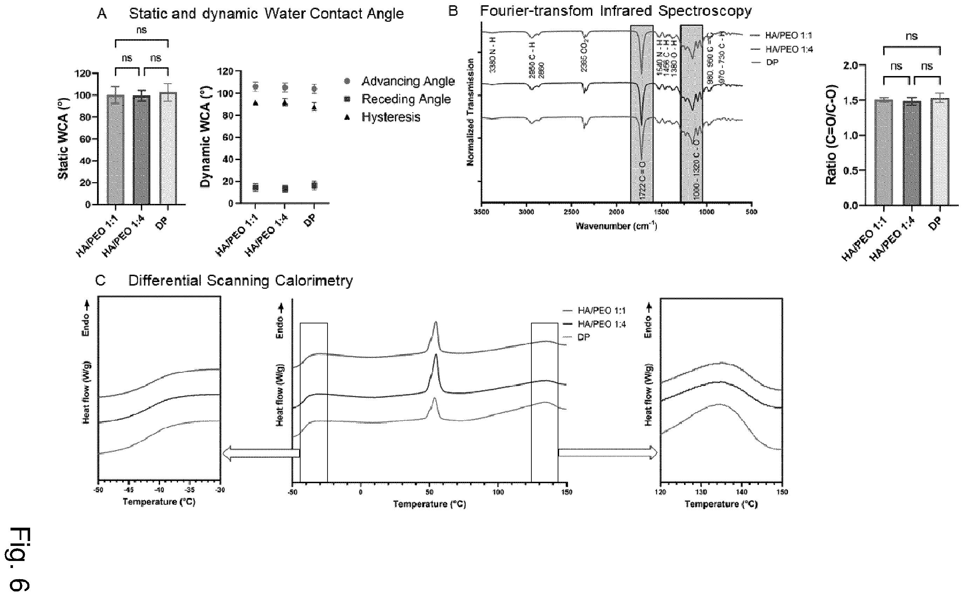

- Fig. 6: shows an analysis of Water Contact Angles (WCA), Fourier-transform Infrared Spectroscopy (FTIR) and Differential Scanning Calorimetry (DSC) of scaffolds, namely:
**A**) static and dynamic WCA measurements of the inner surface or HA containing scaffolds or pure DP scaffolds. Hysteresis = advancing WCA - receding WCA. Number of tubes / scaffold material n = 3. Number of static WCA measurements / scaffold material n = 50, number of dynamic WCA measurements / scaffold material n = 18 for advancing WCA and n = 17 for receding WCA. An ordinary one-way-ANOVA for each surface was performed (Tukey's multiple comparisons test) to determine significant median differences applying 95 % confidence interval. p-values ≤ 0.05 were considered significant and denoted with (*), for p-values ≤ 0.01 (**), p-values ≤ 0.001 (***) and for non-significant (ns) was used;
**B**) FTIR absorbance spectra of the HA containing scaffolds compared to pure DP scaffolds using 4 scaffolds per material (n = 4) and performing 3 technical replicates for every sample (measurements per material n = 12). Spectra have been vertically shifted for visual clarity. For FTIR spectra of each scaffold, see Figure S1. Ratio of prominent peaks assigned to C=O and C-O junctions is shown in in the bar chart;
**C**) DSC thermograms of different scaffolds and enlarged areas of glass transition temperature (Tg) on the left and melting point (Tm) on the right. For DSC thermograms of each scaffold, see Figure S2. Number of tubes / scaffold material n = 4. Thermograms have been vertically shifted for visual clarity.

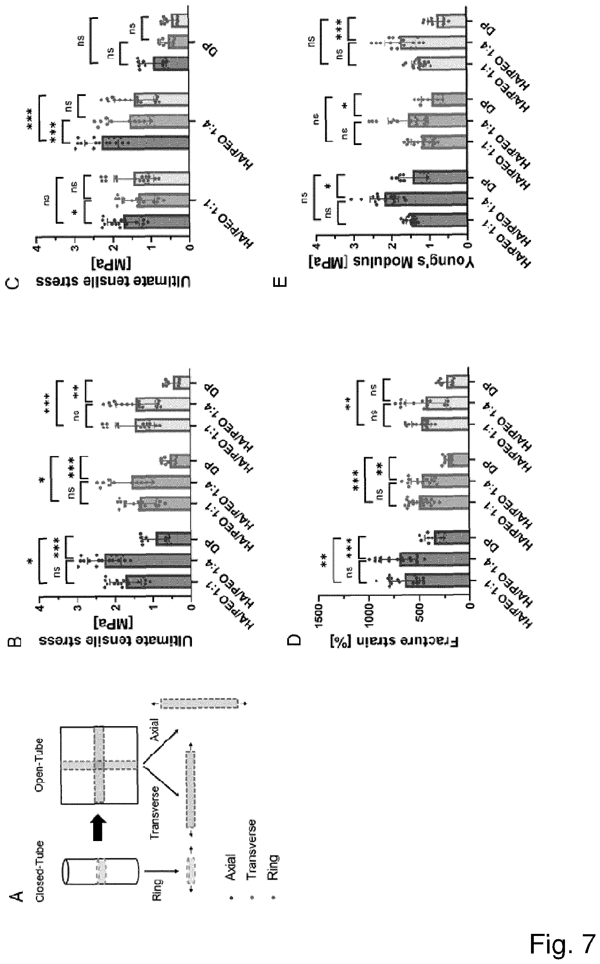

- Fig. 7: shows mechanical properties of HA containing tubes and pure DP tubes, namely:
**A**) Experimental assessment for axial and transverse measurements;
**B**) Ultimate tensile stress (UTS) comparing the different materials;
**C**) UTS comparing the different measurements in the same material;
**D**) Fracture strain, and
**E**) Young's modulus comparing the different scaffold materials; data are shown as mean and SD with individual values; tubes per HA containing material n = 3 and pure DP tube n = 2, performing 6 measurements per sample; normal distribution was checked with D'Agostino & Pearson (α = 0.05); Kruskal-Wallis test was performed to compare the different tubes (Fig. 7B, 7D, 7E) and the different methods for pure DP in Fig. 7C; due to given normal distribution within HA containing tubes in Fig. 7C, an ordinary one-way-ANOVA was carried out to compare different behavior (Tukey's multiple comparisons test) applying 95 % confidence interval; in all graphical layouts p-values ≤ 0.05 were considered significant and denoted with (*), for p-values ≤ 0.01 (**), p-values ≤ 0.001 (***) and for non-significant (ns) was used;

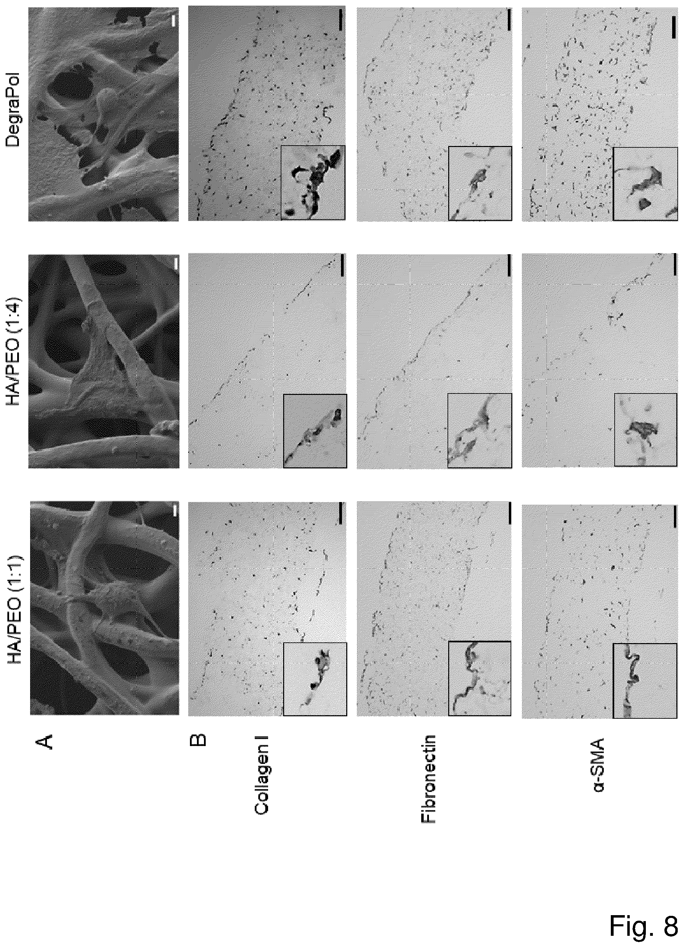

- Fig. 8: shows a qualitative analysis of tenocytes seeded on scaffolds, namely:
**A)** SEM images of tenocytes seeded on different scaffold materials. For better visibility, tenocytes are colored in red. Scale bars: 3 µm;
**B)** Immunohistochemical staining of tenocyte seeded scaffolds 100× magnified. Scale bars: 100 µm. Integrated small images show tenocytes at higher magnification (zoom into 400× magnified image);

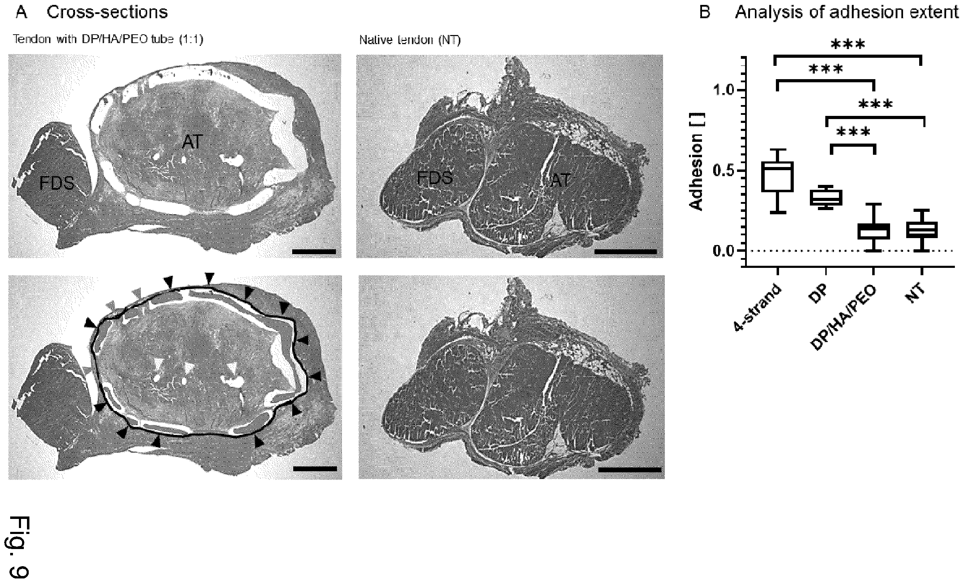

- Fig. 9: shows an assessment of adhesion, namely:
**A**) Picrosirius Red stained cross-sections of rabbit Achilles tendon (AT) treated with a DP/HA/PEO tube 3 weeks post-surgery and of native rabbit AT. Flexor digitorum superficialis (FDS) is also visible. The DP/HA/PEO tube is indicated in blue color. Black arrows label non-adhesive tissue, while green arrows mark adhesion to surrounding tissue. Yellow arrows in the middle of AT are pointing at holes caused by the 4 -strand suture. Scale bar = 1 mm;
**B**) Percentage of adhesion extent calculated based on histology, dividing length of adhesion (green line) by total tendon circumference (black line) in A. Results from 4 -strand sutured tendons with or without DP implant respectively, were taken from an earlier publication [29] for direct comparison. Data are shown as box and whisker plots with interquartile range and 95 % confidence interval. Rabbits used per treatment: n = 6 for 4strand sutured tendons and DP treated tendons; n = 3 for DP/HA/PEO treated tendons and native tendons (NT). Analyzed sections per treatment: n = 11 for 4 -strand sutured tendons and DP treated tendons; n = 46 for DP/HA/PEO treated tendons and n = 32 for native tendons (NT). Kruskal-Wallis test was performed applying 95 % confidence interval. In all graphical layouts p-values ≤ 0.05 were considered significant and denoted with (*), for p-values ≤ 0.01 (**), p-values ≤ 0.001 (***).

### Detailed description of the invention

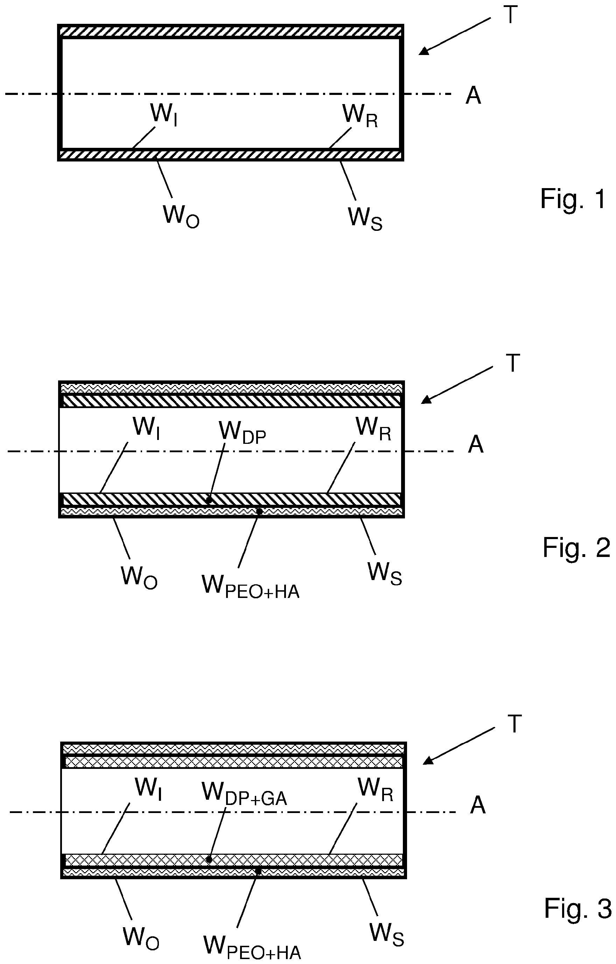

The device for repair surgery of cylindrical organs, particularly ruptured tendons, as shown in Fig. 1 is configured as a tubular sheath T made of a biocompatible and biodegradable polymer. The tubular sheath T is approximately cylindrically shaped along a longitudinal axis A. It comprises an elastic fiber mesh formed by electrospinning of said polymers and has an inner wall surface W_{I} and an outer wall surface Wo substantially parallel thereto. One of said wall surfaces - in the presently shown case it is the inner wall surface W_{I} - is comparatively rough W_{R} whereas the other one of said wall surfaces - in the presently shown case it is the outer wall surface Wo - is comparatively smooth Ws . The configuration shown in Fig. 1 is the one to be used for the intended surgical application. As explained further above, this configuration is obtained from a freshly produced electrospun tubular sheath by everting the same, i.e. by switching the inner and outer side thereof. The outer layer comprises fibers of polyethylene oxide and fibers of hyaluronic acid.

In the embodiment shown in Fig. 2, the inner layer consists of neat degrapol. In the embodiment shown in Fig. 3, the inner layer consists of neat degrapol including a therapeutic agent GA.

### Example 1

A 2-layered implant was made by electrospinning. It was characterized with respect to surface characteristics (fiber thickness and wall thick-ness by SEM). In addition, the water contact angle has been determined. Moreover, it was implanted around a fully transected rabbit Achilles tendon that was sutured. After 3 weeks, histological analysis of the adhesion extent revealed negligible adhesion - on the same level as non-treated naive Achilles tendons, which is a highly promising result. In the same model without such a tube, we had approximately 40 - 50 % of adhesion. The rabbit Achilles tendon full transection model is used because the tendons have approximately the same strength as typical human tendons of the hand.

It was shown in vivo that the tubular implant acts anti-adhesive in an excellent manner (0-5 % of adhesion as the natural tendon). There was no adhesion formation, which was caused by the anti-adhesive effects that are attributed to the bio-lubricant hyaluronic acid in the layer facing the surrounding tissue.

### Example 2

### 1. Introduction

Tendons consist of dense viscoelastic, fibrous connective tissue and transfer muscle force to bones enabling joint motion and stabilization. They are hypovascular [1, 2] and hypocellular with a low metabolic activity leading to a limited natural healing capacity, frequently resulting in the formation of fibrovascular scar tissue and inferior mechanical and functional properties [3]. Accidents or overuse in daily life or in sports, but also age-related degeneration or adiposity [4], may be risk factors for tendon disease affecting four millions new patients per year worldwide [1, 5-7]. Tendons heal in three overlapping phases: Immediately after injury, an inflammatory response occurs including intrinsic and extrinsic pathways lasting for about 3 - 7 days [8]. In the following repair phase, the injury site becomes hypercellular characterized by an increase in myofibroblasts and deposition of new extracellular matrix (ECM) material. After about 6 weeks, the remodeling phase begins replacing the randomly orientated collagen III fibers by collagen I and orientating them in longitudinal structures [9-11]. Although the third phase can last up to one year, quality of the healed tendon remains inferior due to still decreased fiber orientation and formation of scar tissue leading to worse biomechanical properties. Several animal models have confirmed these findings [12, 13] documenting that repaired tendons reach only 40-70 % of uninjured tendon strength [14-16]. Re-rupture of the repaired tendons, joint stiffness and adhesion formation are common clinical problems leading to secondary surgeries, persisting functional disability and impairments in patients daily lives [17].

Thanks to animal models and cell-culture based experiments tendon healing processes are better understood but in the clinics surgery and rehabilitation are still the gold standard so far [10, 18]. Rabbit Achilles tendon (AT) and human hand flexor tendons show similar biomechanical properties with nearly the same ultimate failure load [19, 20] and although not having a synovial sheath rabbit AT are inclined to develop adhesion formation [20, 21]. This makes the rabbit AT a good model to gain knowledge for improving hand injury treatments, an important field for plastic surgeons as hand injuries represent 20 % of all injuries treated in emergency [6, 22].

An effective method to reduce adhesion formation after surgery is the use of physical barriers, limiting the contact between the tendon and the surrounding tissue but allowing cytokines, growth factors and metabolic waste to pass without disturbing tendon movement, mechanical properties and the healing process [8, 18, 23]. Synthetic and biological biomaterials such as polymers based on hydroxy-butyrate and ε-caprolactone, a biodegradable and biocompatible polyester urethane [21, 24], chitosanbased scaffolds [25], decellularized extracellular matrix (ECM) [26] or Type I collagen-based scaffolds [18, 23] have been used in animal models amongst others. The polyester urethane DegraPol^{®}15 (DP) utilized in this study has been shown to be a good scaffold material for tenocytes [27] and previous work demonstrated that cellular response in a rabbit full laceration AT model was not impaired while adhesion formation could be reduced by about 20 % [28, 29]. In addition, the elasticity of the electrospun DP tube is an advantage for the surgery because the material has a high strain at break compared to other commonly used materials. Furthermore, electrospun scaffolds have an ECM-like morphology with a wide range of pore diameter distribution and a high surface area-to-volume ratio and are often used as carriers for therapeutic agents [30-33]. As adhesion extent in rabbit AT full laceration model has been shown to be reduced with pure DP implants [29] we aimed to improve the electrospun implant by adding hyaluronic acid (HA) to reduce adhesion formation even further.

Hyaluronic acid is a major component of the ECM, playing essential roles in cell signaling, wound healing, tissue regeneration and matrix organization [34, 35]. It is an important lubricating component of the synovial fluid and supplies the tendon with nutrients [36]. Macrophages and fibroblast-like cells synthesize HA in the synovial membrane [37, 38] and its production increases after injuries, which improves cell viability and cell proliferation, and reduces inflammation [34, 39-41]. Thanks to the biocompatible and biodegradable properties, HA is often applied in medical therapies and experimental studies [34, 35, 42, 43]. Injections of HA have shown positive effects regarding tendon maturity, strength, stiffness and joint function in animal models [41, 44, 45] possibly induced by a reduced inflammation stage. Due to the lubricating properties and the presence in the synovial fluid the application of HA is frequently used with the aim to reduce adhesion after surgery and collected data show that a repeated administration of sodium hyaluronate may improve the postoperative active finger motion in human [46].

Hyaluronic acid is an unbranched glycosaminoglycan with hydrophilic properties [34]. Effects of HA depend on its molecular weight ranging from 5 × 10⁴-2 × 10⁸ Da [33] and its concentration influencing signaling pathways and differential macrophage activation [34, 39, 47] but also viscosity and viscoelasticity [34]. High molecular weight (HMW) HA (> 10⁶ Da) has been shown to have immunosuppressive, anti-angiogenic and anti-inflammatory effects [48], while low molecular weight (LMW) HA shows opposite effects [49, 50]. The balance between HA synthesis provided by hyaluronan synthases and HA degradation by haluronidases and oxidative damage caused by reactive oxygen species (ROS) [34, 35] is therefore very important. Hyaluronic acid in combination with autografts and allografts is a promising treatment option in wound healing [32, 51], but both methods have limitations like the unavailability of healthy donor sites or possible immune rejection. Thanks to the 3D nanostructure of electrospun scaffolds [52], their application has been shown to be a good alternative treatment reducing adhesion as well [29]. Additionally, usage of HA in electrospun scaffolds showed positive effects regarding bacterial biofilm formation and adherence, and proliferation of cells [31, 32]. Rabbit tenocytes supplemented with HMW HA exhibited a general downregulation of matrix markers *in vitro,* and collagen crosslinking enzyme LOX, suggesting that HA *in vivo* will not provoke a fibrotic reaction but rather prevents adhesion [53].

Thanks to its biodegradability (half-life in blood stream of 2 - 5 minutes [54]), its biocompatibility and biological functions, there is a big interest in HA containing electrospun scaffolds. However, due to high viscosity, high surface tension, and high electrical conductivity, electrospinning of HA is challenging [55]. Solvents are often used to decrease viscosity and surface tension to enable electrospinning but HA like other polymers is not soluble in volatile organic solvents [33]. The association of HA with other well processable polymers like poly(vinyl alcohol) (PVA) or polyethylene oxide (PEO) enables HA electrospinning while maintaining its biological benefits [56].

Summarily, many experimental approaches, including the use of growth factors, bioactive agents [57], stem cells, tissue engineering and optimized suture techniques [58] are undertaken with the goal to enhance tendon repair. Despite encouraging progress in the last decades, the long-term clinical results still need to be improved [59, 60].

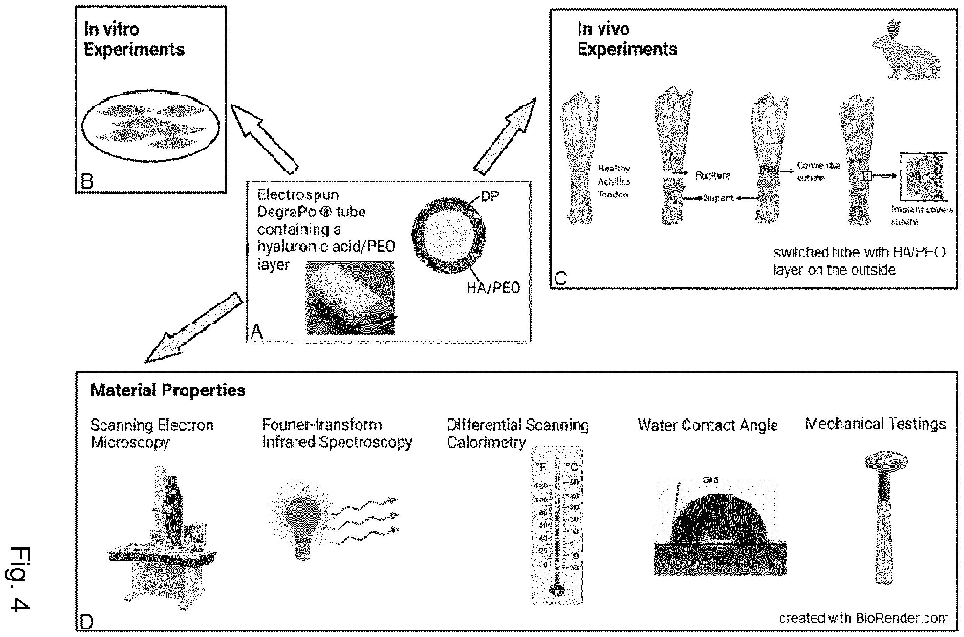

In this study, we produced an electrospun DegraPol^{®} tube containing a layer of HA/PEO [61] in the ratio 1:1 or 1:4 respectively (Fig. 4A), with the aim to use HA as biolubricant and improve the anti-adhesive effect of the DegraPol^{®} tube used in earlier studies [21, 28, 29]. Tubes with a HA/PEO ratio of 1:1 were switched before surgery and applied in a full laceration AT model [20], resulting in an implant with a HA containing layer facing the surrounding tissue (Fig. 4C). Three weeks after implantation, the adhesion extent of the extracted tendons was analyzed using Picrosirius Red staining and results were compared with tendons treated with non-bioactive DP tubes and native tendons. In order to investigate adhesion and proliferation of rabbit tenocytes on the new scaffolds, cells were seeded on the scaffolds and analyzed with Scanning electron microscopy (SEM) and light microscope after immunostaining. Proliferation dynamics were assessed, using alamarBlue assay (Fig. 4B). As HA was the first time added to DP electrospun scaffolds, material properties were analyzed with SEM, Fourier-transformed infrared Spectroscopy (FTIR), Differential Scanning Calorimetry (DSC), Water Contact Angle (WCA), and by testing the mechanical qualities (Fig. 4D).

### 2. Material and methods

### 2.1. Materials

For electrospinning DegraPol^{®}15 (DP), a biodegradable polyester urethane block copolymer, was kindly provided by Ab Medica, Italy. High molecular weight hyaloronic acid (HA, 1.01 - 1.8 MDa) was ordered from Lifecore Biomedical (Lifecore Biomedical #HA 15M, Chaska, USA). Chloroform (#132950), 1,1,1,3,3,3-hexa fluoro-2-propanol (HFP) (#105228), polyethylene glycol (PEG) (in average 35'000 g/mol) (#81310) and polyethylene oxide (PEO) (in average 600'0000 g/mol) (#182028) were purchased from Sigma-Aldrich (Buchs, Switzerland). Solutions for electrospinning were filled in a 5 mL glass syringe (Huberlab, #3.7102.33, Aesch, Switzerland).

For cell culture, gentamycine (# L0011), Ham's F12 (# L0135-500) and FBS (# S1830-500) were bought from Biowest (Nuaillé, France) and amphotericin B (# P06-01100) from Pan Biotech (Aidenbach, Germany). Penicillin/streptomycin (# 15140122) and GlutaMAX^{™} (# 35050038) were delivered from ThermoFisher scientific (Basel, Switzerland) and phosphate buffered saline (PBS) (# D8537) from Sigma-Aldrich (Merck, Buchs, Switzerland). Cells were cultured in tissue cultre plates from Corning (PrimariaTM, Corning, New York, NY, USA) and alamarBlue^{™} cell viability assay (# DAL1100) from Invitrogen (ThermoFisher Scientific, Basel, Switzerland) was carried out in 12-well plates from Sigma-Aldrich (Merck, Buchs, Switzerland, # 665180) and 96-well plates (# 92096) from Techno Plastic Products AG (TPP) (Trasadingen, Switzerland).

For immunocytochemical staining an Autostainer Link48 (DAKO, Basel, Switzerland) was used. Target retrieval solution (# GV80411) and washing buffer (# K800721) were ordered from DAKO (DAKO, Baar, Switzerland). Hydrogen peroxide H₂O₂ was bought from Sigma-Aldrich (Merck, Buchs, Switzerland, # 1.07209.0250) and Pertex from HistoLab (Biosystems, Muttenz, Switzerland #00801-EX) was used to fix coverslips. For Collagen I staining a goat polyclonal primary antibody was bought from abeam (abeam, Cambridge, UK, # ab24821; 1:100 dilution), horse anti-goat IgG antibody (H+L) peroxidase was ordered from Biozol (Biozol, Eching, Germany, # VEC-PI-9500) and horse serum from AdipoGen (AdipoGen, Liestal, Switzerland, # VC-S-2000) was used for blocking. For Fibronectin staining a mouse monoclonal primary antibody was purchased from Sigma-Aldrich (Sigma-Aldrich, Merck, Buchs, Switzerland, # F0791; 1:200 dilution) and for α-SMA staining a mouse monoclonal primary antibody from DAKO was applied (Agilent Technologies, Basel, Switzerland, # IR611; 1:2 dilution). For Fibronectin and α-SMA staining a peroxidase labelled polymer conjugated to goat anti-mouse immunoglobulins from DAKO was used (Agilent Technologies, Basel, Switzerland, # K4001) and samples were blocked with goat serum (AdipoGen, Liestal, Switzerland, # VC-S-1000).

To chemically dry scaffolds seeded with tenocytes for SEM, 1,1,1,3,3,3-hexamethyldisilazan (HMDS) (#3840.3) from Carl Roth AG (Arlesheim, Switzerland) was used.

### 2.2. Methods

### 2.2.1. Solution preparation for electrospinning

A 12 wt % DP polymer solution was prepared by dissolving DP in a mixture of chloroform/hexafluoropropylene (HFP) (80:20 wt/wt) at room temperature the day before electrospinning. HA/PEO solutions (2 wt %) were prepared at 1:1 and 1:4 weight ratios and dissolved in MilliQ water, under stirring at 500 rpm for 48 hours at room temperature. PEG (30 wt %) was dissolved in chloroform at room temperature and used for electrospinning the next day.

### 2.2.2. Electrospinning

Electrospinning was carried out with an in-house assembled electrospinning device consisting of a DC high voltage supply (Glassman High Voltage Inc., High Bridge, NJ, US), a spinning head with a blunt end and a stainless steel tube (1 mm inner diameter and 0.3 mm wall thickness, Angst & Pfister AG, Zürich, Switzerland). The spinning head was fixed on a transporter and connected via a Teflon hose with a syringe pump (SP210cZ, WPI, Germany). The solutions were filled in a 5 mL glass syringe and the electrospun fibers were collected on a metal rod with a diameter of 6 mm and a length of 450-550 mm fixed to a rotary motor (Euro Star B rotary motor, IKA Labortechnik) rotating with 500 rpm. Flow rate was set at 1 mL/h for all solutions. For PEG and DP solutions, 12.5 kV and a working distance of 18.5 cm were used while layers containing HA/PEO were produced with 20 kV and a working distance of 14 cm. The electrospinning was performed at room temperature (22-25 °C) with an air humidity between 21-35 % for PEG and DP, and 21-26 % for HA/PEO solutions. In all tubes, the first layer consisted of a thin PEG layer facilitating the detachment of the tube from the metal rod using 50 % ethanol. The Teflon hose was rinsed with chloroform and dried with air between using different solutions. For the *in vivo* experiment, a first layer of HA/PEO was electrospun followed by a pure DP layer and tubes were flipped before implantation. For the *in vitro* experiments three layered tubes were produced with a DP layer in the middle flanked by a HA/PEO layer on each side to ensure cell seeding on a HA/PEO surface.

### 2.2.3. Scanning electron microscopy (SEM)

Electrospun scaffolds were mounted on metal stubs using conducting double-sided tape and were sputter coated with 10 nm platinum (safematic CCU-010, Zizers, Switzerland). For each material three samples (n = 3) were analyzed by SEM (Zeiss Gemini 450, Feldbach, Switzerland) at an accelerating voltage of 5 kV using the secondary electron detector. Three different locations per scaffold were captured at 700× magnification and pictures were analyzed with ImageJ (1.53e/Java 1.8.0_172 (64-bit)). To determine fiber thickness a diagonal was drawn on the picture and fibers crossing the line were measured. To get pore sizes both diagonals were used and pores on the surface were measured. Images from tenocyte seeded scaffolds were captured at an accelerating voltage of 1.5 kV using the secondary electron detector at 5000× magnification and analyzed qualitatively.

### 2.2.4. Fourier-transform infrared spectroscopy (FTIR)

Fourier transformed infrared spectroscopy was performed on a Varian 640 Fourier Transform Infrared Spectrometer (FTIR) equipped with a Golden Gate-diamond ATR with temperature control. For the spectra, 64 scans were averaged collected in a wavenumber range of 600-4000 cm⁻¹ at a resolution of 4 cm⁻¹. Four scaffolds per material (n = 4) were used and three regions of each scaffold were scanned. For visual presentation of the data, normalization to the C=O peak at 1720 cm⁻¹ was performed using GraphPad Prism 9 for Windows (GraphPad Software, San Diego, California USA). To compare the different scaffolds, the ratio between the C=O peak at 1720 cm⁻¹ and the C-O peak at 1175 cm⁻¹ was calculated. Corresponding bonds were assigned by comparing the peaks to IR-spectrum table (Merck KGaA, Darmstadt, Germany).

### 2.2.5. Differential scanning calorimetry (DSC)

Thermal analysis of the samples was carried out using a differential scanning calorimeter, DSC2500 (TA Instruments). Four scaffolds per material (n = 4) in a weight range of 3 to 15 mg were analyzed. Two heating cycles with an intermediate cooling cycle were performed from -90 °C to 170 °C with a heating and cooling rate of 10 °C/min. To compare the different samples, only the second heating cycle was taken into consideration. The glass transition point and the phase transition enthalpy were calculated with the software of the DSC (TA Instruments TRIOS v5.1.1.46527).

### 2.2.6. Static and dynamic water contact angles (WCA)

Static and dynamic water contact angles on scaffold surfaces were measured by a video-based optical contact angle measuring instrument (OCA 35 Dataphysics, Germany). For static WCA a drop of 5 µl Milli-Q water was put on the scaffold surface according to reported WCA measurements [62]. The angles on both drop sites were measured for three scaffolds per material (n = 3) and measurements were repeated 5-10 times per scaffold. The calculated angle mean was defined as static WCA.

For dynamic WCA the advancing and receding WCAs were measured and hysteresis was calculated by substracting receding WCA from advancing WCA values [63]. To determine the advancing WCA an initial drop of 5 µl Milli-Q water was positioned on the scaffold and was filled continuously with water. Angles were measured when the drop baseline on the scaffold jerkily increased. The receding angle was measured the same way but removing water from the 5 µl water drop. To measure the contact angle, a video of the process was taken, the left and right angles were measured using ImageJ (1.53e/Java 1.8.0_172 (64-bit )) and averages were calculated. Three samples were used per material (n = 3) and three measurements were carried out per sample.

### 2.2.7. Mechanical tests

To measure the stress-strain curve of the different materials a uniaxial load test machine (Zwick Z010, 20 kN load-cell, testXpert III; Zwick/Roell, Ulm, Germany) was used and a strain rate of 10 mm/min until failure was applied for all specimens. To perform axial and transverse measurements, samples were cut into rectangular pieces of 2 mm × 18 mm and clamped to a gauge length of 10 mm. For ring measurements, 2 mm specimens were cut and clamped to a gage length of 8 mm. Ultimate tensile stress (UTS), fracture strain and the Young's modulus were determined as the peak stress to failure, strain at failure, and the slope of a linear fit of the stress-stain curve up to 20 % strain respectively. Calculation of mechanical parameters was performed with MATLAB (Release 2021a, The MathWorks, Inc., Natick, MA, USA). Stress was defined as force over specimen cross sectional area and strain as the percent change in length from the gage length. For the HA containing tubes, 3 tubes per material were used and 6 measurements per tube were carried out. For the pure DP tubes, 2 samples with 6 repetitions were measured.

### 2.2.8. Cell isolation

Rabbit tenocytes were isolated from ATs of three New Zealand White rabbits using the cell migration method (Approval by the veterinary office of Canton Zurich, reference number ZH 080/2021; 33530). Briefly, tendons were extracted from the animals and washed with PBS supplemented with 200 µg/mL gentamicin and 2.5 µg/mL amphotericin B. Tendons were cleaned from the surrounding tissue and the central part of the tendons was cut into very small pieces (< 2 mm) and washed three times in PBS buffer. Afterwards, multiple tissue pieces were placed into a tissue culture plate and a drop of cell culture medium was added onto each tissue piece (Ham's F12, 10 % FBS, 100 U/mL penicillin, 100 µg/mL streptomycin and 1 % GlutaMAX). Tissues were allowed to attach onto the cell culture plates for 2 hours at 37 °C and 5 % CO₂ before adding 10 mL of cell culture media into each plate. The plates with the tissues were not moved for the first 5 days, to decrease tissue detachment upon plate movement and to allow cells to start migrating out from the tissues. The first medium change was done after 5 days, and subsequently, the culture medium was changed every third day. After approximately 2 weeks, tissue pieces were removed from the plates, and cells were allowed to proliferate for 1 week more before cryopreservation. Cryopreserved rabbit tenocytes were thawed, resuspended in culture medium and cultured at 37 °C and 5 % CO₂ with media being changed every second day. Tenocytes between passages 2 and 4 (P 2-4) were used for all experiments. The same procedure has been reported before [64].

### 2.2.9. Cell seeding and proliferation assay on scaffolds

Pure DP scaffolds and DP scaffolds containing HA/PEO layers were sterilized with UV for 30 minutes on each side. Half of the scaffolds were shortly wetted in 50 % ethanol afterwards, rinsed three times with water and washed once with medium before cells were seeded on the scaffold surface in a 12-well plate. Circles of 5 mm diameter were punched out of all different scaffolds and tenocytes from three donors (P 2 - P 3, 5 × 10⁵ cells in 10 µL medium) were seeded on top and allowed to settle at 37 °C and 5 % CO₂ for about 3 h before adding 1 mL cell culture media to each well. The culture media was exchanged every third day and the experiment was carried out in technical duplicates. Cell proliferation was determined on day 3, 7 and 14 by alamarBlue^{™} cell viability assay. Thereafter, the scaffolds were transferred into a 96-well plate and incubated with 100 µL 1:10 diluted alamarBlue^{™} solution for 4 h before excitation wavelength of 530 nm and emission wavelength of 590 nm were measured using a Cytation 5 imaging reader (BioTEk, Agilent Technologies AG, Basel, Switzerland). After measurement, scaffolds were washed once with medium before adding 100 µL culture medium for further cultivation. Empty wells were filled with PBS to prevent dehydration. After measurement on day 14, scaffolds were fixed in 4 % paraformaldehyde (PFA) for further immunostaining and in 0.5 % glutaraldehyde / 3 % PFA for further SEM preparation, and stored in the fridge.

### 2.2.10. Histological analysis and sample preparation for SEM analysis of cell seeded scaffolds

Tenocyte seeded scaffolds were embedded in paraffin according to commonly established protocols and cut into 3 µm thick slices before deparaffinization with xylene and subsequent rehydration. Immunocytochemical staining for collagen I, fibronectin and α-SMA was carried out with standardized protocols, using Autostainer Link48. Shortly, after antigen retrieval samples were exposed to 3 % H₂O₂ for 10 min. After washing with washing buffer, samples were blocked with serum for 30 min and primary antibody was added for 1 h. After the next washing step, samples were treated with HRP for 20 min., washed again and exposed to DAB for 10 min. Then samples were stained with hematoxylin for 10 min. and rinsed with washing buffer before they were transferred into water and dehydrated with ethanol. Slides were imaged for qualitative analysis at 100×, 200× and 400× magnification using a confocal microscope (Leica AF 6000B).

For SEM preparation, tenocyte seeded scaffolds were washed once in PBS and dehydrated in a series of ascending concentration of ethanol (30 %, 50 %, 70 %, 80 %, 95 % (5 min each step) and 100 % (2 times 10 min). Subsequently, scaffolds were chemically dried in HMDS/ethanol mix (1:3, 1:1, 3:1) and pure HMDS, each step for 15 min). HMDS was allowed to evaporate overnight, and the samples were mounted on SEM stubs. For SEM imaging see preceding section 2.2.3.

### 2.2.11. In vivo implantation

For the implantation of HA/PEO tubes in a ratio 1:1, 3 female New Zealand White rabbits, aged 12-16 weeks, specific pathogen free (SPF), were used (Charles River, Research Models and Services, Germany). The animals were housed, maintained and fed as previously described [20] and acclimatized to their environment for 2 weeks before surgery. Ethical approval for the experiments was achieved from the veterinary office of Zurich, Switzerland (reference numbers ZH 080/2021; 33530). The full transection of the Achilles tendon 2 cm above the calcaneus, followed by a 4 -strand Becker suture was carried out as described earlier [20]. The tubes were sterilized with H₂O₂ (plasma sterilization) before implantation and flipped over the wound so that the HA containing layer was facing the surrounding tissue. Afterwards the wound was closed with a running suture (using a USP 6.0 polypropylene fibre) and a well-padded cast was applied with an angle of 180° at the ankle. The rabbits got a Durogesic Matrix patch after surgery (Janssen-Cilag AG, Switzerland) with 4.2 mg Fentanyl per patch to provide analgesia for about 72 h with 25 µg/h Fentanyl. The rabbits were euthanized three weeks later in deep anaesthesia (100 mg/kg Ketamine and 4 mg/kg Xylazine) with 80 mg/kg Pentobarbital (Esconarkon ad us. vet., Switzerland) and the tendons were removed. Surgery was performed on one hind leg while the counter hind leg was not treated (NT) and served as control. The extracted tendons were immediately frozen and stored at -20 °C in a gauze moistened with 0.9 % NaCl-solution.

### 2.2.12. Histological analysis of repaired tendons

The tendons were thawed overnight at 4 °C and warmed to RT before they were dehydrated and embedded in paraffin according to commonly established protocols. Cros sections of 5 µm in the wound region (perpendicular to the Achilles tendon) were deparaffinized with xylene and rehydrated prior histological staining with Hematoxylin-Eosin (H&E), Alcian Blue (AB) and Picrosirius Red (PS) according to commonly established procedures. To quantify adhesion extent, PS-stained sections in five subsequent cross-sections separated by 2.0 mm as described by Tan *et al.* (2010) were analyzed at 8× magnification (Leica EZ4D microscope, Switzerland). To calculate the percentage of adhesion, the length of the contact region of the tendon with the surrounding tissue and the whole tendon perimeter were determined with synedra view software (version 22.0.0.12), before dividing the length of the contact region by the length of the total perimeter.

### 2.2.13. Statistical analysis

Data were analyzed with GraphPad Prism 9 (Version 9.4.0 GraphPad Software Inc., California, US). To test normal distribution Kolmogorov-Smirnov test was applied. For normally distributed data or results with a high number of data (n > 30), a one-way analysis of variance (ANOVA with Tukey's multiple comparisons test) was used. Not normally distributed results were analyzed with the nonparametric Kruskal-Wallis test. P-values ≤ 0.05 were considered significant and denoted with an asterisk within graphs (*p ≤ 0.05; **p ≤ 0.01; ***p ≤ 0.001, and (ns) for non-significant). Calculations were performed in Excel (2016 (16.0.5332.1000) MSO (16.0.5278.1000) 32-bit).

### 3. Results

### 3.1. Microfibrous scaffold characterization using scanning electron microscope images (SEM)

Double layered DP scaffolds containing a layer of HA and PEO in a ratio of 1:1 or 1:4, respectively, were successfully produced using the protocol from Vitkova *et al.* [61] and Evrova *et al.* [57] for the DP layer. Hyaluronic acid containing scaffolds were compared to pure DP scaffolds using SEM images (Fig. 5A), showing a network of random fibers with a diameter of about 6 µm in average in every material (HA/PEO (1:1) 6.1 µm ± 2.5 µm, HA/PEO (1:4) 6.7 µm ± 3.0 µm, DP 6.7 µm ± 2.8 µm) (Fig. 5B). More than half of the fibers had a diameter within 5-10 µm, about a third of the fibers showed smaller diameters and only about 10 % of the fibers possessed a diameter larger than 10 µm (Fig. 5B). The significant differences in fiber thickness can be explained by the large number of analyzed fibers (n = 411 for HA/PEO (1:1), n = 737 for HA/PEO (1:4) and n = 1178 for DP). Pore sizes of scaffolds containing HA were significantly larger than in pure DP scaffolds; with HA/PEO (1:1) 14.5 µm ± 6.3 µm, HA/PEO (1:4) 15.8 µm ± 6.8 µm and DP 9.6 µm ± 6.2 µm (Fig. 5C). Nearly 60 % of the pores in HA containing scaffolds showed a diameter of 10-20 µm, three times more pores than in DP scaffolds in which about 70 % of the pores were smaller than 10 µm (Fig. 5C).

### 3.2. Static and dynamic water contact angle (WCA) measurements

The static WCA provides information about the wettability of materials and is lower than 90° for hydrophilic surfaces, whereas hydrophobic surfaces show WCAs higher than 90°. Although HA is highly hydrophilic, static WCAs of HAcontaining scaffolds were only slightly lower than values of DP scaffolds and were not significantly different with values of about 100°, classified as moderate hydrophobic material (HA/PEO (1:1) = 100.1° ± 7.5°, HA/PEO (1:4) = 99.5° ± 5.1°, DP = 107.6° ± 4.7°) (Fig. 6A).

Contact angle hysteresis (CAH) is calculated by subtracting the receding contact angle from the advancing contact angle. High CAH values are attributed mainly to surface heterogeneity and roughness [63, 65, 66]. For the dynamic measuring, all different materials show low receding WCAs and high advancing WCAs leading to high hysteresis (Fig. 6B), with minimally lower values for pure DP material (HA/PEO (1:1) 91.6° ± 0.8°, HA/PEO (1:4) 91.9° ± 3.4°, DP 87.8° ± 3.6°).

### 3.3. Analysis of Fourier-transform Infrared Spectroscopy (FTIR)

Fourier-transform Infrared Spectroscopy was assessed for the different scaffold material showing very similar spectra of all meshes. This result was expected because the amount of electrospun HA or PEO respectively, was very low (HA/PEO (1:1) about 5 % w/v each; HA/PEO (1:4) about 1.2 % w/v HA and 4.8 % w/v PEO). All spectra express a prominent peak at 1722 cm⁻¹ assigned to the carbonyl group (C=O stretching) and a larger region at 1300-1000 cm⁻¹ associated with symmetric and asymmetric stretching vibrations of C-O. Ratios of C=O bonds to C-O bonds were comparable for all tested scaffolds as shows the right graph of Fig. 6B. The peaks at 2940 cm⁻¹ and 2860 cm⁻¹ correspond to stretching vibrations of -CH₂ and CH₃ groups, while the small peak at about 3380 cm⁻¹ is associated with NH stretching. In contrast, the peak at 2360 cm⁻¹ results from free CO₂ in the device during measurement.

### 3.4. Thermal analysis by Differential Scanning Calorimetry (DSC)

Thermograms to determine glass transition temperature (T_{g}) of amorphous material and melting point (Tₘ) of crystalline components were assessed using DSC [67] (Fig. 6C). Addition of HA and PEO did not change the typical thermal pattern of DP with a T_{g} at about -40 °C and a Tₘ at around 130 °C. The prominent endothermic peak at about 56 °C corresponds to Tₘ of PEG, used as first layer to facilitate removal of the tube from the metal, or to PEO respectively, enabling electrospinning of HA. Polyethylene oxide and PEG are both ethylene oxide polymers differing only in their molecular weight (PEG in average 35'000 g/mol; PEO in average 600'000 g/mol).

### 3.5. Mechanical properties

Mechanical properties such as ultimate tensile stress (UTS), fracture strain and Young's Modulus were tested in the axial and transverse direction of the different scaffolds to determine possible mechanical differences due to the presence of HA and PEO (Fig. 7). Transverse direction measurements were performed either in a ring piece or in a rectangle of an opened tube (Fig. 7A). For UTS, values of HA/PEO containing tubes were two to three times higher compared to pure DP tubes, independent of the direction and method; while results of the two HA containing tubes with different HA/PEO concentrations showed comparable results with slightly higher values in the axial direction for the ratio 1:4 (Fig. 7B) (mean values axial measurements [MPa]: HA/PEO (1:1) 1.73 ± 0.41, HA/PEO (1:4) 2.29 ± 0.44, DP 0.93 ± 0.35; transverse measurements [MPa]: HA/PEO (1:1) 1.34 ± 0.42, HA/PEO (1:4) 1.56 ± 0.54, DP 0.55 ± 0.18; ring measurements [MPa]: HA/PEO (1:1) 1.46 ± 0.52, HA/PEO (1:4) 1.43 ± 0.52, DP 0.46 ± 0.19). Comparing UTS in the same material, HA/PEO tubes with a ratio of 1:4 showed 1.5 -times higher values in the axial direction than in the transverse direction. For tubes with a ratio of 1:1 only axial compared to rectangular transverse measuring was significantly higher (1.3 -fold) but not compared to values obtained by ring measurements. Axial results of DP tubes were higher than transverse results but not significantly different (Fig. 7C).

Similar results can be observed for fracture strain measurements showing no or only moderate difference between HA containing tubes but achieving two times higher values compared to pure DP tubes (Fig. 7D) (mean values axial measurements [%]: HA/PEO (1:1) 647 ± 153, HA/PEO (1:4) 670 ± 177, DP 350 ± 91; transverse measurements [%]: HA/PEO (1:1) 495 ± 108, HA/PEO (1:4) 468 ± 108, DP 208 ± 39; ring measurements [%]: HA/PEO (1:1) 471 ± 93, HA/PEO (1:4) 427 ± 191, DP 220 ± 70).

In the Young's modulus DP tubes show the lowest values, but only the HA containing tube in a ratio 1:4 reaches significantly higher values compared to pure DP material, about 1.5 -times higher for axial and rectangular transverse measuring and double as high results for ring measurements (Fig. 7E). Consistently, comparing results from both HA containing tubes results are not significantly different although values from tubes with the ratio 1:4 are in tendency higher (mean values axial measurements [MPa]: HA/PEO (1:1) 1.49 ± 0.13, HA/PEO (1:4) 2.2 ± 0.40, DP 1.45 ± 0.37; transverse measurements [MPa]: HA/PEO (1:1) 1.19 ± 0.28, HA/PEO (1:4) 1.56 ± 0.52, DP 0.92 ± 0.28; ring measurements [MPa]: HA/PEO (1:1) 1.26 ± 0.18, HA/PEO (1:4) 1.76 ± 0.40, DP 0.79 ± 0.23).

### 3.6. Cell adhesion, ECM production and proliferation of rabbit tenocytes in vitro

Scanning electron microscope pictures of scaffolds pre-treated with 50 % ethanol and fixated on day 3 show that tenocytes are able to attach to the fibers of HA/PEO containing scaffolds and pure DP scaffold. Different shapes and cell morphologies were observed on every scaffold type (Fig. 8A). Immunostainings on 50 % ethanol pre-treated scaffolds, fixated on day 14 and stained with Collagen I, Fibronectin or α-smooth muscle actin (α-SMA) demonstrate that tenocytes are able to invade the scaffolds and to produce ECM (Fig. 8B). For all markers strongest staining was observed on pure DP scaffolds and weakest on HA/PEO in a ratio 1:4. In general, only few cells produced Collagen I while many cells expressed Fibronectin and α-SMA, resulting in a stronger staining. AlamarBlue staining confirms adhesion of the tenocytes to the scaffolds on day 3. On 50 % ethanol pre-treated scaffolds cell seeding results in higher amount of adhered tenocytes compared to not pre-treated scaffolds, but cell viability decreases with time. In contrast, less tenocytes attached to the fibers of not pre-treated scaffolds, but tenocytes were able to proliferate between day 3 and day 7 before cell number decreases.

### 3.7. Effect of HA/PEO tubes on adhesion formation in rabbit full laceration AT model

The effect of HA/PEO containing DP tubes with a ratio 1:1 in the full laceration AT model was evaluated by histological analysis of the near contact region between the tendon and the surrounding tissue 3 weeks after surgery (Fig. 9). Results were compared with native tendons and with earlier results from repaired tendons with or without DP tube implantation [29]. Large areas of non-adhesive tissue can be seen on sections of tendons with a DP/HA/PEO tube and although HA/PEO layer is directed towards the surroundings, anti-adhesive effects can be observed as well on the inner tube layer facing the tendon tissue (Fig. 9A). While pure DP tubes decreased adhesion extent in 4 -strand sutured tendons by about 20 % in comparison with sutured tendons without tube [29], application of HA/PEO containing DP tube reduced adhesion of sutured tendons significantly by about 50 % compared to sutured tendons treated with pure DP implants and reached adhesion levels comparable to native tendons (Fig. 9). Adhesion visible in native tendon is probably a technical artefact caused by the cutting process.

### 4. Discussion

Tendon healing is a long-lasting process, and natural regeneration capacity of tendons is limited due to their hypovascularization, the dense connective tissue, low cell density and slow cell metabolism. In addition, re-rupture and adhesion formation are common clinical problems after surgery [22, 68, 69]. Adhesion results from the generation of fibrotic tissue between the tendon and the surroundings and may impair tendon gliding, leading to joint stiffness and pain in up to 30 % of flexor tendon injuries [11]. The detailed mechanism of adhesion formation is still unclear, but persistent inflammation is known to activate fibrogenesis leading to an excessive accumulation of ECM components, such as collagen and alpha smooth muscle actin (α-SMA) [70, 71].

Post-operative mobilization with moderate mechanical load [72] has been shown to reduce adhesion extent, but also drugs [72], gowth factors [59], and physical barriers have been used for that purpose [29, 73]. Because adhesion correlates with compromised tendon gliding capacity, application of biolubricants, for instance HA, is another interesting therapeutic approach [74] as HA is biocompatible and a key component of synovial fluid, ensuring good lubrication of synovial joints [75]. Hyaluronic acid is involved in several important biological processes, such as wound healing and tissue regeneration, cell signaling or matrix reorganization, but its effect depends on many factors like concentration and molecular weight. While high molecular weight (HMW) HA has been shown to have immunosuppressive, anti-angiogenic and anti-inflammatory effects, low molecular weight (LMW) HA shows opposite outcomes [34].

Promising results were obtained from the combination of mechanical with biological approaches by adding anti-adhesive HA into scaffold materials, although no such product is available for routine use in clinical practice so far [73]. Electrospun tubes of polycaprolactone alone [76] or in combination with chitosan [32] or acrylate endcapped urethane precursor [77], Zein [31, 78] or PEO [79] have been used in combination with HA, showing promising results, but demonstrating that further investigation is necessary. Another successfully electrospun and commercially available material is DegraPol, a biodegradable, biocompatible and surgeon friendly polyester urethane. No adverse reactions of the tendon tissue following tube implantation in a full laceration rabbit AT model was observed [21, 24], but adhesion extent was reduced by about 20 % [20, 29].

The aim of this study was to improve the anti-adhesive properties of DP tubes [29, 57] making use of the reported positive anti-adhesive effects of HA in a rabbit full laceration model. Previous results, using HMW HA on tenocytes *in vitro,* have demonstrated that HMW HA did not influence cell proliferation and might have a positive effect on gene expression of some relevant ECM markers. In consequence, it should not improve fibrotic reaction *in vivo,* but rather avoid adhesion formation [53]. As DP scaffolds containing HA were not fabricated before, the new material was characterized by SEM, static and dynamic WCA, DSC thermograms and FTIR, mechanical properties, and by checking attachment and proliferation of rabbit tenocytes on HA containing DP scaffolds.

Analysis of SEM images showed a network of fibers with a diameter of about 6 µm in average in pure DP scaffolds as well as in DP/HA/PEO scaffolds independent of the HA/PEO ratio (Fig. 5B) confirming values of DP fibers published by Rieber *et al.* [80]. Many factors like humidity, temperature, viscosity or jet stability of the spinneret influence electrospinning outcome, which may lead to differently pronounced morphology of the electrospun material despite constant voltage and flow rate. A possible result of such influences is the high variability of fiber thickness, leading to a large standard deviation, which is most prominent in HA/PEO containing DP tubes with a ratio 1:4. Although average of fiber diameters in every material is similar statistical analysis showed significant differences between the scaffolds because of the large number of analyzed fibers (HA/PEO (1:1) n = 411; HA/PEO (1:4) n = 737; DP n = 1178). Consistently, distribution of fiber diameter is similar for all meshes with more than half of the fibers having diameters ranged from 5-10 µm, about a third of the fibers showing smaller diameters and only about 10 % of the fibers possessing a diameter larger than 10 µm (Fig. 5B). In contrast to the comparable results for fiber thickness, pore sizes of scaffolds containing HA/PEO were significantly larger than in pure DP scaffolds (Fig. 5C) with nearly 60 % of the pores having a diameter of 10-20 µm in HA/PEO containing scaffolds, whereas 70 % of the pores in DP scaffolds were smaller than 10 µm (Fig. 5C). As voltage, flow rate and rotating speed of the metal rod were kept constant, the larger pore diameters have to be attributed to modified viscosity, conductivity or surface tension of the HA/PEO containing solution [81]. Pore size in HA/PEO containing tubes with a ratio 1:4 showed slightly larger pore diameters than pores in tubes with ratio 1:1, consistent with results from Chen et al [82]. This demonstrates that increasing amount of PEO increased pore size in the electrospun PCL scaffolds, possibly influenced by the higher viscosity of the more concentrated PEO solutions. Although pore size has been shown to play a role in cell attachment [79] in our study tenocyte adhesion is similar between the different scaffolds. This might be explained by the fact, that pore sizes of the different scaffolds are similar despite the statistical difference. AlamarBlue experiments demonstrate that tenocytes attach to every scaffold material, but a decreasing cell number over time was visible, although control cells on plastic proliferated well. Therefore, limited permeability for nutrition, oxygen and metabolic waste might reduce cell viability in the scaffold [79] consistent with results from Torres-Sanchez *et al.* [83] demonstrating that small pores favor cell attachment while proliferation is higher in materials with larger pore size.

Mechanical strength and stretchability are important parameters of implants, influencing their handling during tendon surgery, which is often hampered by limited space at the operation site. Formation of fiber thickness, fiber orientation, and crosslinking, as well as nanotopographical structures like pores, ridges or grooves induce changes in the mechanical properties. Formation of such features is affected by many factors like polymer concentration and viscosity, applied voltage and flow rate, nature of solvent, but also temperature and humidity during electrospinning, amongst other [84]. Moreover, addition of supplementary substances, for instance proteins or platelet-rich plasma (PRP), influences mechanical properties showing reduced UTS with increased protein molecular weight [85] or formation of thicker fibers in the case of PRP [76]. However, fiber thickness is not the only parameter influencing mechanical strength as supplementation with HA/PEO in both ratios increased mechanical strength compared to pure DP scaffolds significantly but did not influence fiber thickness. Ultimate tensile stress of HA/PEO containing material was two to three times higher than in pure DP tubes. This is consistent with the data obtained by Chen *et al.* showing that addition of HA/iboprufen (IBU) increased mechanical properties of the scaffold without influencing fiber thickness [79]. Comparing the UTS of the same material, axial values were in general higher than transverse values most prominently pronounced in the 1:4 HA/PEO scaffold, which demonstrates the anisotropy of the scaffolds. Lower mechanical strength in the transverse direction might be a result of tertiary structures like ridges and grooves, acting as co-defects described by Evrova *et al.* [81]. As wall thickness has been shown to influence mechanical properties [76] the thinner wall diameter of the analyzed DP tubes (400 µm ± 57 µm), compared to the DP tubes used by Evrova *et al.* (500-800 µm) [81], might explain the slightly different results eventually caused by less pronounced formation of nanotopographic structures. Wall thickness of HA/PEO containing DP tubes have about the same thickness as the pure DP tube (HA/PEO (1:1) 487 µm ± 53 µm, HA/PEO (1:4) 436 µm ± 63 µm), demonstrating the impact of HA/PEO on material properties. Pore size influences mechanical properties as well, depending on the scaffold material and the range of their size. For instance, in titanium scaffolds having pore sizes from 45-500 µm larger pore sizes reduced mechanical strength [86], but in hyaluronan-collagen scaffolds having pores ranging from 300-500 µm larger pores increased mechanical rupture stresses and Young's Moduli [87]. Although pore size in the HA/PEO containing scaffolds was in average larger and mechanical properties were enhanced compared to DP scaffolds, influence of pore size on mechanical strength might be irrelevant, as pores are similar in every material and effect is probably rather caused by the addition of HA and PEO. As for UTS, fracture strain of HA/PEO containing tubes showed two times higher values than DP tubes, demonstrating the great effect of a HA/PEO layer that influences mechanical strength and stretchability of the material. In addition, results from Young's modulus setting the UTS in relation to the length change were significantly lower for DP tubes compared with 1:4 ratio HA/PEO tubes but not compared with 1:1 ratio tubes, indicating the important role of PEO regarding mechanical scaffold properties. Polyethylene oxide, a water-soluble synthetic polymer [88] improving electrospinning processability of aqueous solutions [78], has been shown to improve mechanical properties of commonly used biodegradable polymer electrospun scaffolds [89] and to result in increased UTS with increasing PEO concentrations [90]. In summary, addition of HA/PEO increased the mechanical properties of the scaffolds significantly, which facilitates the application of the implants during tendon surgery when material stretchability is needed mainly in the transverse direction. However, mechanical strength of the implants is too low to be used as reinforcement of injured tendons after surgery or in tendinopathy patients. For comparison, UTS of rabbit AT are in the range of 30 MPa and Young's modulus about 100 MPa [21] showing similar strength as human flexor tendons, while HA/PEO containing scaffolds showed for both, UTS and Young's modulus, values in the range of 1.5-2 MPa. However, as HA/PEO containing tubes are envisioned to reduce adhesion extent after surgery and not to act as tendon reinforcement, their low mechanical strength compared to tendon tissue is not relevant for this purpose.

An important property of scaffolds used *in vitro* or *in vivo* is the wettability of the surface characterized by determination of the static water contact angle (WCA), influencing cellular attachment and proliferation as well as bacterial adhesion. Many factors are influencing the static WCA such as surface energy and roughness [66]. Surfaces with a static WCA lower than 90° are considered as hydrophilic, whereas hydrophobic surfaces show higher WCAs than 90°. Superhydrophobic materials with WCA between 150° and 180° have been shown to reduce bacterial adhesion and prevent biofilm formation [91]. Although HA and PEO are hydrophilic, static WCAs of HA/PEO containing scaffolds were only slightly lower than values from DP scaffolds and were not significantly different with values of about 100. They were therefore classified as moderately hydrophobic materials (HA/PEO (1:1) = 100.1° ± 7.5°, HA/PEO (1:4) = 99.5° ± 5.1°, DP = 107.6° ± 4.7°) (Fig. 6A). Another interesting characteristic is the difference between the advancing and the receding contact angle defined as contact angle hysteresis (CAH), which is attributed mainly to surface heterogeneity and roughness [63, 65, 66]. In the dynamic contact angle measurements, all different materials showed low receding angles, leading to a high hysteresis (Fig. 6B), indicating that scaffold materials are heterogeneous, which was confirmed by SEM images (Figure 1). The roughness of the surfaces might explain the hydrophobic characteristics as contact angles can be increased with the surface roughness and air pockets at the water-scaffold interface [92].

Fourier-transform Infrared Spectroscopy was used to check possible changes in the transmission energy by the addition of HA and PEO, giving insights into the chemical composition of the tested materials (Fig. 6B). As expected, FTIR spectra of all tested scaffolds look very similar because the amount of electrospun HA or PEO respectively, is very low (HA/PEO (1:1) about 5 % w/v each; HA/PEO (1:4) about 1.2 % w/v HA and 4.8 % w/v PEO) and therefore typical peaks of HA or PEO are superimposed by peaks of DP [32, 67, 80]. This result confirms data from Hu *et al.* showing that a low amount of supplemented substances does not change FTIR spectra [93]. The FTIR peaks are consistent with data from earlier DP measurements (Evrova, 2020); with a prominent peak at 1722 cm⁻¹ assigned to the carbonyl group (C=O stretching) and a larger region at 1300 - 1000 cm⁻¹ associated with symmetric and asymmetric stretching vibrations of C-O junctions, both caused by ester bonds of the polyester urethane polymer DP [94, 95]. Ratios of C=O bonds to C-O bonds were similar for all tested scaffolds (Fig. 6B) as described in chapter 3.3. In summary, the FTIR spectra indicate that no new bonds, between the DP polymer and HA or PEO respectively, were formed as peak pattern from pure DP scaffolds are identical to patterns of HA and PEO containing scaffolds. However, same peak intensity of pure DP tubes and HA/PEO/DP tubes as well as the missing of typical HA and PEO peaks suggest that relative amount of HA and PEO is small, and therefore these components might not be detectable with FTIR.

To evaluate a possible change in thermal behavior by adding HA and PEO into electrospun DP scaffolds, the second DSC heating cycle was analyzed to determine the glass transition temperature (T_{g}), at which amorphous, solid material gets rubbery and viscous, and the melting point (Tₘ) of crystalline components [67] (Fig. 6C). As expected, HA and PEO supplementation did not change the typical thermal pattern of DP with a T_{g} at about -40 °C and a Tₘ at around 130 °C consistent with published data [57, 80]. The prominent endothermic peak at about 56 °C corresponds to Tₘ of PEG or PEO, respectively. As PEO is used to enable HA electrospinning, it is present in the HA containing tubes, whereas the peak in the pure DP scaffolds results from incomplete washing of PEG that was used as first layer to facilitate taking off the tube from the metal rod [80]. Though, the peak at 56 °C is smaller in pure DP scaffolds than in HA/PEO containing scaffolds and peak in HA/PEO scaffolds in a ratio 1:4 is slightly higher than in scaffolds with a ratio 1:1, it cannot be distinguished between PEG contamination and PEO integration in HA/PEO containing DP tubes. Interestingly, rests of PEG are detectable with DSC and also PEO contents might be seen in DSC thermograms, as corresponding peaks are moderately higher in DP/HA/PEO scaffolds. This stands in contrast to FTIR spectra, where no such differences could be observed.

Scanning electron microscope images show that tenocytes are able to attach to the fibers of the different scaffold materials and immunohistochemical labeling for Collagen I, Fibronectin and α-SMA proof that tenocytes are able to express ECM (Fig. 8). In general, stronger staining was observed for tenocytes seeded on HA/PEO in the ratio 1:1 than in the ratio 1:4, eventually due to the positive effect of HA on matrix organization, cell viability and cell activity [34, 40]. In contrast to Fibronectin and α-SMA, less cells expressed Collagen I consistent with gene expression results from Miescher *et al.* [53], showing a decreased Collagen I expression in rabbit tenocytes using HMW HA (the same HA that was used here). AlamarBlue assay confirm attachment of tenocytes to scaffold fibers but show reduced cell viability over two weeks possibly due to limited permeability of nutrition, oxygen and metabolic waste within the scaffold [79].

The rabbit animal model has often been used to examine tendon healing and to enable translation of therapeutic assessments into clinical care [96]. For instance, cellular response towards DP implants [24], ultrasound studies [97] and adhesion formation [29, 98] were studied using this model. The effect of HA/PEO containing DP tubes with a ratio 1:1 was evaluated regarding adhesion prevention in the rabbit full laceration AT model by histological analysis of the near contact region between the tendon and the surrounding tissue 3 weeks after surgery (Fig. 9). Results were compared with native tendons and with earlier results from repaired tendons with or without DP tube implantation [29]. Negligible adhesion towards the surrounding tissue was visible on Picrosirius Red stained cross-sections of HA/PEO/DP tube-treated AT, comparable to native AT from the control contralateral legs (Fig. 9A), which was substantiated quantitatively (Fig. 9B). Impressively, while pure DP tubes decreased adhesion extent in 4 -strand sutured tendons by about 20 % in comparison with sutured tendons without tube [29], application of HA/PEO containing DP tube reduced adhesion significantly by about 50 % compared to sutured tendons treated with pure DP implants, reaching an adhesion extent similar to native tendons, i.e. negligible adhesions (Fig. 9B). However, anti-adhesive effects of HA could be observed as well on the inner tube layer of pure DP, facing the tendon tissue. This side effect may be caused by HA diffusion and the strong lubricating properties of HA even in small amounts. The anti-adhesive effect of both tube sides might be reduced by adding more DP layers, eventually with incorporated growth factors [80], to enhance tendon regeneration as has been previously shown by Evrova *et al.* [57].

### 5. Conclusion

We have produced an electrospun DP implant with a second layer of HA/PEO with the aim to reduce adhesion formation in a rabbit full laceration Achilles tendon model even more than by the pure DP implant (20 % reduction). The results from the 3 weeks *in vivo* assessment demonstrate that adhesion extent was reduced significantly compared to a pure DP implant, reaching a level comparable to healthy tendons. *In vitro* studies have shown that rabbit tenocytes are able to attach on every scaffold material, but cell viability decreased over a time period of 2 weeks, probably due to the limited permeability for nutrition, oxygen and metabolic waste within the scaffold. The novel scaffolds were analyzed in detail, including fiber thickness and pore size (SEM), FTIR, DSC, static and dynamic WCA and mechanical tests. No major differences could be observed between pure DP tubes and HA/PEO containing DP tubes. Only the mechanical properties of HA/PEO/DP tubes were increased. This study demonstrates that an additional HA/PEO layer on an electrospun DP scaffold shows promising potential in reducing adhesion formation after tendon surgery. Therefore, these novel bi-layered implant show great potential for a clinical application in the future.

### References

1. Tang, J.B.e.a., Basic FGF or VEGF gene therapy corrects insufficiency in the intrinsic healing capacity of tendons. Sci. Rep., 2016. 6(20643).
2. Linderman, S.W., et al., Cell and Biologic-Based Treatment of Flexor Tendon Injuries. Oper Tech Orthop, 2016. 26(3): p. 206-215.
3. Galatz, L.M., et al., Tendon regeneration and scar formation: The concept of scarless healing. J Orthop Res, 2015. 33(6): p. 823-31.
4. Gaida, J.E., J.L. Cook, and S.L. Bass, Adiposity and tendinopathy. Disabil Rehabil, 2008. 30(20-22): p. 1555-62.
5. Clayton, R.A. and C.M. Court-Brown, The epidemiology of musculoskeletal tendinous and ligamentous injuries. Injury, 2008. 39(12): p. 1338-44.
6. de Jong, J.P., et al., The incidence of acute traumatic tendon injuries in the hand and wrist: a 10-year population-based study. Clin Orthop Surg, 2014. 6(2): p. 196-202.
7. Ren, Z., et al., Instantaneous self-healing and strongly adhesive self-adaptive hyaluronic acidbased hydrogel for controlled drug release to promote tendon wound healing. Int J Biol Macromol, 2023. 242(Pt 2): p. 125001.
8. Graham, J.G., et al., Biologic and mechanical aspects of tendon fibrosis after injury and repair. Connect Tissue Res, 2019. 60(1): p. 10-20.
9. Sharma, P. and N. Maffulli, Biology of tendon injury: healing, modeling and remodeling. J Musculoskelet Neuronal Interact, 2006. 6(2): p. 181-90.
10. Voleti, P.B., M.R. Buckley, and L.J. Soslowsky, Tendon healing: repair and regeneration. Annu Rev Biomed Eng, 2012. 14: p. 47-71.
11. Titan, A.L., et al., Flexor Tendon: Development, Healing, Adhesion Formation, and Contributing Growth Factors. Plast Reconstr Surg, 2019. 144(4): p. 639e-647e.
12. Juncosa-Melvin, N., et al., Effects of mechanical stimulation on the biomechanics and histology of stem cell-collagen sponge constructs for rabbit patellar tendon repair. Tissue Eng, 2006. 12(8): p. 2291-300.
13. Walden, G., et al., A Clinical, Biological, and Biomaterials Perspective into Tendon Injuries and Regeneration. Tissue Eng Part B Rev, 2017. 23(1): p. 44-58.
14. Loiselle, A.E., et al., Remodeling of murine intrasynovial tendon adhesions following injury: MMP and neotendon gene expression. J Orthop Res, 2009. 27(6): p. 833-40.
15. Proctor, C.S., D.W. Jackson, and T.M. Simon, Characterization of the repair tissue after removal of the central one-third of the patellar ligament. An experimental study in a goat model. J Bone Joint Surg Am, 1997. 79(7): p. 997-1006.
16. Carpenter, J.E., et al., Rotator cuff defect healing: a biomechanical and histologic analysis in an animal model. J Shoulder Elbow Surg, 1998. 7(6): p. 599-605.
17. Tang, J.B., Clinical outcomes associated with flexor tendon repair. Hand Clin, 2005. 21(2): p. 199-210.
18. Veronesi, F., et al., Evaluation of a new collagen-based medical device (ElastiCo®) for the treatment of acute Achilles tendon injury and prevention of peritendinous adhesions: An in vitro biocompatibility and in vivo investigation. J Tissue Eng Regen Med, 2020. 14(8): p. 1113-1125.
19. Goodman, H.J. and J. Choueka, Biomechanics of the flexor tendons. Hand Clin, 2005. 21(2): p. 129-49.
20. Meier Bürgisser, G., et al., Rabbit Achilles tendon full transection model - wound healing, adhesion formation and biomechanics at 3, 6 and 12 weeks post-surgery. Biol Open, 2016. 5(9): p. 1324-33.
21. Meier Biirgisser, G., et al., Prevention of peritendinous adhesions using an electrospun DegraPol polymer tube: a histological, ultrasonographic, and biomechanical study in rabbits. Biomed Res Int, 2014. 2014: p. 656240.
22. Chartier, C., et al., Tendon: Principles of Healing and Repair. Semin Plast Surg, 2021. 35(3): p. 211-215.
23. Zhao, H., et al., Collagen membrane alleviates peritendinous adhesion in the rat Achilles tendon injury model. Chin Med J (Engl), 2013. 126(4): p. 729-33.
24. Buschmann, J., et al., Synthesis, characterization and histomorphometric analysis of cellular response to a new elastic DegraPol® polymer for rabbit Achilles tendon rupture repair. J Tissue Eng Regen Med, 2015. 9(5): p. 584-94.
25. Chen, E., et al., An asymmetric chitosan scaffold for tendon tissue engineering: In vitro and in vivo evaluation with rat tendon stem/progenitor cells. Acta Biomater, 2018. 73: p. 377-387.
26. Lipar, M., et al., Extracellular matrix supports healing of transected rabbit Achilles tendon. Heliyon, 2018. 4(9): p. e00781.
27. Saad, B., et al., Multiblock copolyesters as biomaterials: in vitro biocompatibility testing. J Mater Sci Mater Med, 1997. 8(8): p. 497-505.
28. Buschmann, J., et al., Cellular response of healing tissue to DegraPol tube implantation in rabbit Achilles tendon rupture repair: an in vivo histomorphometric study. J Tissue Eng Regen Med, 2013. 7(5): p. 413-20.
29. Bürgisser, G.M., et al., Electrospun tube reduces adhesion in rabbit Achilles tendon 12 weeks post-surgery without PAR-2 overexpression. Sci Rep, 2021. 11(1): p. 23293.
30. S. Sahoo, L.T.A., J.C. Goh, S.L. Toh, Growth factor delivery through electrospun nanofibers in scaffolds for tissue engineering applications,. J. Biomed. Mater, 2010. Res. A 93: p. 1539-1550.
31. Figueira, D.R., et al., Production and characterization of polycaprolactone- hyaluronic acid/chitosan- zein electrospun bilayer nanofibrous membrane for tissue regeneration. Int J Biol Macromol, 2016. 93(Pt A): p. 1100-1110.
32. Chanda, A., et al., Electrospun chitosan/polycaprolactone-hyaluronic acid bilayered scaffold for potential wound healing applications. Int J Biol Macromol, 2018. 116: p. 774-785.
33. Castro, K.C., M.G.N. Campos, and L.H.I. Mei, Hyaluronic acid electrospinning: Challenges, applications in wound dressings and new perspectives. Int J Biol Macromol, 2021. 173: p. 251-266.
34. Fallacara, A., et al., Hyaluronic Acid in the Third Millennium. Polymers (Basel), 2018. 10(7).
35. Kobayashi, T., T. Chanmee, and N. Itano, Hyaluronan: Metabolism and Function. Biomolecules, 2020. 10(11).
36. Abate, M., C. Schiavone, and V. Salini, The use of hyaluronic acid after tendon surgery and in tendinopathies. Biomed Res Int, 2014. 2014: p. 783632.
37. Tercic, D. and B. Bozic, The basis of the synovial fluid analysis. Clin Chem Lab Med, 2001. 39(12): p. 1221-6.
38. Balazs, E.A., et al., Hyaluronic acid in synovial fluid. I. Molecular parameters of hyaluronic acid in normal and arthritis human fluids. Arthritis Rheum, 1967. 10(4): p. 357-76.
39. Aya, K.L. and R. Stern, Hyaluronan in wound healing: rediscovering a major player. Wound Repair Regen, 2014. 22(5): p. 579-93.
40. Osti, L., et al., Hyaluronic acid increases tendon derived cell viability and collagen type I expression in vitro: Comparative study of four different Hyaluronic acid preparations by molecular weight. BMC Musculoskelet Disord, 2015. 16: p. 284.
41. Li, H., Y. Chen, and S. Chen, Enhancement of rotator cuff tendon-bone healing using bone marrow-stimulating technique along with hyaluronic acid. J Orthop Translat, 2019. 17: p. 96-102.
42. Kaux, J.F., A. Samson, and J.M. Crielaard, Hyaluronic acid and tendon lesions. Muscles Ligaments Tendons J, 2015. 5(4): p. 264-9.
43. Loebel, C., et al., Fabrication of cell-compatible hyaluronan hydrogels with a wide range of biophysical properties through high tyramine functionalization. J Mater Chem B, 2017. 5(12): p. 2355-2363.
44. Honda, H., et al., Hyaluronic Acid Accelerates Tendon-to-Bone Healing After Rotator Cuff Repair. Am J Sports Med, 2017. 45(14): p. 3322-3330.
45. Liang, J.I., et al., The effect of tenocyte/hyaluronic acid therapy on the early recovery of healing Achilles tendon in rats. J Mater Sci Mater Med, 2014. 25(1): p. 217-27.
46. Murray, E., et al., Effectiveness of Sodium Hyaluronate and ADCON-T/N for the Prevention of Adhesions in Hand Flexor Tendon Surgery: A Systematic Review and Meta-Analysis. J Hand Surg Am, 2022. 47(9): p. 896.e1-896.e20.
47. Rayahin, J.E., et al., High and low molecular weight hyaluronic acid differentially influence macrophage activation. ACS Biomater Sci Eng, 2015. 1(7): p. 481-493.
48. Vassallo, V., et al., Unsulfated biotechnological chondroitin by itself as well as in combination with high molecular weight hyaluronan improves the inflammation profile in osteoarthritis in vitro model. J Cell Biochem, 2021. 122(9): p. 1021-36.
49. D'Agostino, A., et al., Is molecular size a discriminating factor in hyaluronan interaction with human cells? Carbohydr Polym, 2017. 157: p. 21-30.
50. Gao, Y., et al., A Low Molecular Weight Hyaluronic Acid Derivative Accelerates Excisional Wound Healing by Modulating Pro-Inflammation, Promoting Epithelialization and Neovascularization, and Remodeling Collagen. Int J Mol Sci, 2019. 20(15).
51. Karabekmez, F.E. and C. Zhao, Surface treatment of flexor tendon autograft and allograft decreases adhesion without an effect of graft cellularity: a pilot study. Clin Orthop Relat Res, 2012. 470(9): p. 2522-7.
52. Smith, L.A. and P.X. Ma, Nano-fibrous scaffolds for tissue engineering. Colloids Surf B Biointerfaces, 2004. 39(3): p. 125-31.
53. Miescher, I., et al., Impact of High-Molecular-Weight Hyaluronic Acid on Gene Expression in Rabbit Achilles Tenocytes In Vitro. Int J Mol Sci, 2022. 23(14).
54. Reed, R.K., et al., Removal rate of [3H]hyaluronan injected subcutaneously in rabbits. Am J Physiol, 1990. 259(2 Pt 2): p. H532-5.
55. Snetkov, P., et al., Hyaluronan-Based Nanofibers: Fabrication, Characterization and Application. Polymers (Basel), 2019. 11(12).
56. Memic, A., et al., Latest Progress in Electrospun Nanofibers for Wound Healing Applications. ACS Appl Bio Mater, 2019. 2(3): p. 952-969.
57. Evrova, O., et al., Elastic and surgeon friendly electrospun tubes delivering PDGF-BB positively impact tendon rupture healing in a rabbit Achilles tendon model. Biomaterials, 2020. 232: p. 119722.
58. Buschmann, J., et al., Small hook thread (Quill) and soft felt internal splint to increase the primary repair strength of lacerated rabbit Achilles tendons: biomechanical analysis and considerations for hand surgery. Clin Biomech (Bristol, Avon), 2011. 26(6): p. 626-31.
59. Docheva, D., et al., Biologies for tendon repair. Adv Drug Deliv Rev, 2015. 84: p. 222-39.
60. Leong, N.L., et al., Tendon and Ligament Healing and Current Approaches to Tendon and Ligament Regeneration. J Orthop Res, 2020. 38(1): p. 7-12.
61. Vítková, L., et al., Electrospinning of Hyaluronan Using Polymer Coelectrospinning and Intermediate Solvent. Polymers (Basel), 2019. 11(9).
62. Hu, M., et al., Nanozymes in Nanofibrous Mats with Haloperoxidase-like Activity To Combat Biofouling. ACS Appl Mater Interfaces, 2018. 10(51): p. 44722-44730.
63. Eral, H.B., 't Mannetje, D.J.C.M., and Oh, J.M., Contact angle hysteresis: a review of fundamentals and applications. Colloid and 663 Polymer Science, 2013. 291(2): p. 247-260.
64. Evrova, O., et al., Supporting Cell-Based Tendon Therapy: Effect of PDGF-BB and Ascorbic Acid on Rabbit Achilles Tenocytes in Vitro. Int J Mol Sci, 2020. 21(2).
65. Extrand, C.W. and Y. Kumagai, An Experimental Study of Contact Angle Hysteresis. J Colloid Interface Sci, 1997. 191(2): p. 378-83.
66. Y.C. Jung, B.B., Contact angle, adhesion and friction properties of micro- and nanopatterned polymers for superhydrophobicity. Nanotechnology, 2006. 17: p. 4970 - 4980.
67. Shalumon, K.T., et al., Multi-functional electrospun antibacterial core-shell nanofibrous membranes for prolonged prevention of post-surgical tendon adhesion and inflammation. Acta Biomater, 2018. 72: p. 121-136.
68. Schulze-Tanzil, G.G., et al., Tendon healing: a concise review on cellular and molecular mechanisms with a particular focus on the Achilles tendon. Bone Joint Res, 2022. 11(8): p. 561-574.
69. Parekh, S.G., et al., Achilles: Failed Acute Repair. Foot Ankle Clin, 2022. 27(2): p. 415-430.
70. Chen, S., et al., RelA/p65 inhibition prevents tendon adhesion by modulating inflammation, cell proliferation, and apoptosis. Cell Death Dis, 2017. 8(3): p. e2710.
71. Taylor, S.H., et al., Tendon is covered by a basement membrane epithelium that is required for cell retention and the prevention of adhesion formation. PLoS One, 2011. 6(1): p. e16337.
72. Elliot, D. and T. Giesen, Primary flexor tendon surgery: the search for a perfect result. Hand Clin, 2013. 29(2): p. 191-206.
73. Imere, A., et al., Engineering a cell-hydrogel-fibre composite to mimic the structure and function of the tendon synovial sheath. Acta Biomater, 2021. 119: p. 140-154.
74. Oliva, F., et al., The Impact of Hyaluronic Acid on Tendon Physiology and Its Clinical Application in Tendinopathies. Cells, 2021. 10(11).
75. Marian, M., et al., Exploring the lubrication mechanisms of synovial fluids for joint longevity - A perspective. Colloids Surf B Biointerfaces, 2021. 206: p. 111926.
76. Chen, C.H., et al., Hyaluronic acid/platelet rich plasma-infused core-shell nanofiber membrane to prevent postoperative tendon adhesion and promote tendon healing. Int J Biol Macromol, 2023. 231: p. 123312.
77. Pien, N., et al., Design and development of a reinforced tubular electrospun construct for the repair of ruptures of deep flexor tendons. Mater Sci Eng C Mater Biol Appl, 2021. 119: p. 111504.
78. Yao, C., X. Li, and T. Song, Fabrication of zein/hyaluronic acid fibrous membranes by electrospinning. J Biomater Sci Polym Ed, 2007. 18(6): p. 731-42.
79. Chen, C.T., et al., Ibuprofen-Loaded Hyaluronic Acid Nanofibrous Membranes for Prevention of Postoperative Tendon Adhesion through Reduction of Inflammation. Int J Mol Sci, 2019. 20(20).
80. Rieber, J., et al., Bioactive and Elastic Emulsion Electrospun DegraPol Tubes Delivering IGF-1 for Tendon Rupture Repair. Int J Mol Sci, 2023. 24(12).
81. Evrova, O., et al., Bioactive, Elastic, and Biodegradable Emulsion Electrospun DegraPol Tube Delivering PDGF-BB for Tendon Rupture Repair. Macromol Biosci, 2016. 16(7): p. 1048-63.
82. Chen, Y., G.Z. Tan, and Y. Zhou, Effects of Viscosities and Solution Composition on Core-Sheath Electrospun Polycaprolactone(PCL) Nanoporous Microtubes. Polymers (Basel), 2021. 13(21).
83. Torres-Sanchez, C., et al., The impact of multimodal pore size considered independently from porosity on mechanical performance and osteogenic behaviour of titanium scaffolds. Mater Sci Eng C Mater Biol Appl, 2021. 124: p. 112026.
84. Kumbar, S.G., et al., Electrospun nanofiber scaffolds: engineering soft tissues. Biomed Mater, 2008. 3(3): p. 034002.
85. Lavin, D.M., et al., Effects of protein molecular weight on the intrinsic material properties and release kinetics of wet spun polymeric microfiber delivery systems. Acta Biomater, 2013. 9(1): p. 4569-78.
86. Torres-Sanchez, C., et al., The effect of pore size and porosity on mechanical properties and biological response of porous titanium scaffolds. Mater Sci Eng C Mater Biol Appl, 2017. 77: p. 219-228.
87. Al-Munajjed, A.A., et al., Influence of pore size on tensile strength, permeability and porosity of hyaluronan-collagen scaffolds. J Mater Sci Mater Med, 2008. 19(8): p. 2859-64.
88. Baker, B.M., et al., The potential to improve cell infiltration in composite fiber-aligned electrospun scaffolds by the selective removal of sacrificial fibers. Biomaterials, 2008. 29(15): p. 2348-58.
89. Hodge, J. and C. Quint, The improvement of cell infiltration in an electrospun scaffold with multiple synthetic biodegradable polymers using sacrificial PEO microparticles. J Biomed Mater Res A, 2019. 107(9): p. 1954-1964.
90. Serôdio, R., et al., Ultrasound sonication prior to electrospinning tailors silk fibroin/PEO membranes for periodontal regeneration. Mater Sci Eng C Mater Biol Appl, 2019. 98: p. 969-981.
91. Bartlet, K., et al., Antibacterial activity on superhydrophobic titania nanotube arrays. Colloids Surf B Biointerfaces, 2018. 166: p. 179-186.
92. Salam, A., et al., In-vitro assessment of appropriate hydrophilic scaffolds by coelectrospinning of poly(1,4 cyclohexane isosorbide terephthalate)/polyvinyl alcohol. Sci Rep, 2020. 10(1): p. 19751.
93. Hu, J., et al., Emulsion electrospinning of polycaprolactone: influence of surfactant type towards the scaffold properties. J Biomater Sci Polym Ed, 2015. 26(1): p. 57-75.
94. Asefnejad, A., et al., Manufacturing of biodegradable polyurethane scaffolds based on polycaprolactone using a phase separation method: physical properties and in vitro assay. Int J Nanomedicine, 2011. 6: p. 2375-84.
95. Wang, Y., P. Li, and L. Kong, Chitosan-modified PLGA nanoparticles with versatile surface for improved drug delivery. AAPS PharmSciTech, 2013. 14(2): p. 585-92.
96. Thomopoulos, S., et al., Mechanisms of tendon injury and repair. J Orthop Res, 2015. 33(6): p. 832-9.
97. Buschmann, J., et al., Correspondence of high-frequency ultrasound and histomorphometry of healing rabbit Achilles tendon tissue. Connect Tissue Res, 2014. 55(2): p. 123-31.
98. Tan, V., et al., Effects of nonsteroidal anti-inflammatory drugs on flexor tendon adhesion. J Hand Surg Am, 2010. 35(6): p. 941-7.

## Claims

1. A device for repair surgery of cylindrical organs, particularly ruptured tendons, configured as a tubular sheath (T) made of a mesh of elastic fibers formed by electrospinning biocompatible and biodegradable polymers, said tubular sheath having a Young elasticity modulus of about 0.1 to about 4 MPa and a strain at break of about 50 to about 1'000 %, said tubular sheath having a first, inner wall surface (W_{I}) and a second, outer wall surface (Wo) substantially parallel thereto, said first, inner wall surface being comparatively rough (W_{R}) and said second, outer wall surface being comparatively smooth (Ws),
wherein said tubular sheath (T) comprises a first tubular layer adjacent to said first, inner wall surface and a second tubular layer adjacent to said second, outer wall surface, said first tubular layer being formed of first polymeric fibers and said second tubular layer being formed by second polymeric fibers,
**characterized in that** said first polymeric fibers are formed from degrapol (DP) and that said second fibers are formed from a blend of high molecular weight hyaluronic acid (HA) and polyethylene oxide (PEO).

2. The device according to claim 1, wherein said first polymeric fibers are formed from degrapol (DP) with an admixture of a therapeutic agent (GA) for stimulating regrowth processes of a predetermined cylindrical organ.

3. The device according to claim 1 or 2, wherein said first tubular layer and said second tubular layer each form about one half of the tubular sheath's wall thickness.

4. A method of producing a device according to one of claims 1 to 3, wherein said elastic fibers are formed by solution electrospinning.
